# EUROPEAN PATENT SPECIFICATION

(11) **EP 2 721 046 B1**
(45) Date of publication and mention of the grant of the patent: **31.08.2016**
(21) Application number: 12730686.8
(22) Date of filing: 14.06.2012
(51) Int. Cl.: C07H 21/00, C07H 15/232, C07K 4/00, C07K 14/00

(54) **METHODS FOR CHEMICAL SYNTHESIS OF BIOLOGICALLY ACTIVE COMPOUNDS USING SUPRAMOLECULAR PROTECTIVE GROUPS AND NOVEL COMPOUNDS OBTAINABLE THEREBY**
VERFAHREN ZUR CHEMISCHEN SYNTHESE VON BIOLOGISCH AKTIVEN VERBINDUNGEN MIT SUPRAMOLEKULAREN SCHÜTZENDEN GRUPPEN UND DARAUS ERHALTENE NEUARTIGE VERBINDUNGEN
PROCÉDÉS DE SYNTHÈSE CHIMIQUE DE COMPOSÉS ACTIFS BIOLOGIQUES UTILISANT DES GROUPES PROTECTEURS SUPRAMOLÉCULAIRES ET NOUVEAUX COMPOSÉS POUVANT AINSI ÊTRE OBTENUS

(30) Priority: 14.06.2011 EP 11169761; 20.07.2011 US 201161509584 P
(43) Date of publication of application: 23.04.2014
(73) Proprietor: Rijksuniversiteit Groningen, 9712 CP Groningen (NL)
(72) Inventor: HERRMANN, Andreas, NL-9747 AG Groningen (NL); BASTIAN,Andreas Alexander, NL-9747 AG Groningen (NL); MARCOZZI, Alessio, NL-9747 AG Groningen (NL)
(74) Representative: V.O.
(86) International application number: PCT/NL2012/050415
(87) International publication number: WO 2012/173477

(56) References cited:
- WO-A2-2005/036169
- WO-A2-2008/040792
- RAVI HEGDE ET AL: "Structure-function relationship of novel X4 HIV-1 entry inhibitors-L- and D-arginine peptide-aminoglycoside conjugates", FEBS JOURNAL, vol. 274, no. 24, 1 December 2007 (2007-12-01), pages 6523-6536, XP055018629, ISSN: 1742-464X, DOI: 10.1111/j.1742-4658.2007.06169.x
- MARKUS KAISER ET AL: "Aminoglycoside-Quinacridine Conjugates: Towards Recognition of the P6.1 Element of Telomerase RNA", CHEMBIOCHEM, vol. 7, no. 2, 6 February 2006 (2006-02-06), pages 321-329, XP055018634, ISSN: 1439-4227, DOI: 10.1002/cbic.200500354
- TOK J B-H ET AL: "Aminoglycoside Hybrids as Potent RNA Antagonists", TETRAHEDRON, ELSEVIER SCIENCE PUBLISHERS, AMSTERDAM, NL, vol. 55, no. 18, 30 April 1999 (1999-04-30), pages 5741-5758, XP004163602, ISSN: 0040-4020, DOI: 10.1016/S0040-4020(99)00240-9
- CARRIERE M ET AL: "Inhibition of protein synthesis by amininoglycoside-arginine conjugates", RNA, COLD SPRING HARBOR LABORATORY PRESS, US, vol. 8, no. 10, 1 January 2002 (2002-01-01), pages 1267-1269, XP002990030, ISSN: 1355-8382, DOI: 10.1017/S1355838202029059
- BETAT H ET AL: "Aptamers That Recognize the Lipid Moiety of the Antibiotic Moenomycin A", BIOLOGICAL CHEMISTRY, WALTER DE GRUYTER GMBH & CO, BERLIN, DE, vol. 384, 1 January 2003 (2003-01-01), pages 1497-1500, XP009156411, ISSN: 1431-6730 cited in the application
- LICONG JIANG ET AL: "Saccharide-RNA recognition in an aminoglycoside antibiotic-RNA aptamer complex", CHEMISTRY AND BIOLOGY, CURRENT BIOLOGY, LONDON, GB, vol. 4, no. 1, 1 January 1997 (1997-01-01) , pages 35-50, XP009156477, ISSN: 1074-5521 cited in the application
- WALLIS M G ET AL: "A NOVEL RNA MOTIF FOR NEOMYCIN RECOGNITION", CHEMISTRY AND BIOLOGY, CURRENT BIOLOGY, LONDON, GB, vol. 2, no. 8, 1 August 1995 (1995-08-01), pages 543-552, XP009003123, ISSN: 1074-5521, DOI: 10.1016/1074-5521(95)90188-4 cited in the application

## Description

The invention relates to drug development and synthetic chemistry, in particular to the manufacture of biologically active compounds based on naturally occurring molecules. It also relates to novel biologically active compounds in a substantially pure regioisomeric form.

The fabrication of natural products and their derivatives is an important task for the understanding of drug action and the development of new pharmaceutically active ingredients. However, their generation typically requires extensive multistep organic synthesis, including the protection and deprotection of functional groups within the target molecule^{1,2,3}.

Regioselectively modified multi functionalized compounds and drugs are therefore laborious and costly to synthesize and afford extended synthetic routes. For example, in case of aminoglycoside antibiotics the regioselective derivatization is restricted to only few functional groups and requires more than 20 synthetic steps with overall yields below 5%.

Very recently, protective groups were introduced that rely on non-covalent interactions and are comprised of macrocyclic systems. Reinaud *et al.* applied a Zn^{II}-complex based on a functionalized calix[6]arene for the chemo- and regioselective carbamoylation of the unsymmetrical triamine spermidine⁴. In the second known example, Kohnke *et al.* utilized a calix[4]pyrrole derivative in the selective O-alkylation of 1,6-dihydroxynaphthalene⁵. The application of non-covalent protective groups based on host-guest interactions can be used as tool for the derivatization of natural products and synthetic molecules as an alternative to extensive covalent protection and deprotection protocols.

Unfortunately, known supramolecular protective groups (SPGs) are still characterized by severe limitations. Due to the small cavity size of available calixarenes the approach is so far limited to small guest molecules exhibiting only two chemical equivalent functional groups. Moreover, generalization of the concept which is based on macrocyclic cavities is very difficult due to extensive efforts needed for the molecular design and synthesis of the host system binding to a beforehand defined guest molecule. Thus, the known SPGs require individual design for each target molecule, involve laborious synthesis, lack a general way of manufacturing and are restricted to bind to small molecules with little structural complexity.

The present inventors therefore aimed at overcoming at least one of these drawbacks by the provision of a novel class of non-covalent SPGs. It is demonstrated herein that SPGs based on oligonucleotides (nucleic acid aptamers) or oligopeptides can be used efficiently for the highly chemo- and regioselective transformation (up to 98%) of bioactive molecules with complicated structures (e.g. antibiotics) in very good conversions (59-83%). Many of the shortcomings of previously reported SPGs were overcome due to the fact that SPGs based on oligonucleotides or oligopeptides are (a) capable binding a diverse range of guest molecules with high affinity and specificity ^{6,7,8,9}, (b) can be generated in a well established selection processes¹⁰ and (c) can be produced by automated synthesis. There are no structural limitations of the guest molecule e.g. with respect to size or functionalities. The high impact of SPGs on synthetic chemistry for the generation of several novel drugs, e.g. antibiotic derivatives, was demonstrated while at the same time avoiding costly, time consuming and laborious synthetic routes including conventional protective group chemistry. The facile functionalization of biologically active natural products or synthetic drugs will facilitate studies on structure-properties relationships and provides easy access to a large number of new drug candidates. The present invention is advantageously applied in a strategy to combat the ever-growing problem of antibiotic resistance. In particular, based on the concept disclosed herein it is possible to revive the antibacterial activity of aminoglycosides using structural modifications that can alter the original mode of action. For example, MRSA and VRE are known to exert high level resistance to aminoglycosides.

The scope of the invention is defined by the appended claims. Any further disclosure not part of the claimed scope is only intended for illustrative purpose.

Hence, in one embodiment the invention provides a method for the chemo- and/or regioselective derivatization of a target compound comprising multiple chemically equivalent reactive groups, wherein at least one reactive group is to be derivatized and wherein at least one reactive group is not to be derivatized, the method comprising the steps of a) contacting the target compound with at least one non-covalent protective group under conditions allowing for the formation of a regioselective host-guest complex, wherein the protective group is selected from oligonucleotide aptamers and oligopeptide aptamers (herein also referred to as "aptameric protective group" or "APG") having a selective affinity for the at least one reactive group not to be modified and wherein the protective group has no affinity for the at least one reactive site to be modified; followed and/or accompanied by b) chemical derivatization of the target compound. Preferably, the host-guest complex is formed prior to the chemical derivatization. Optionally, the derivatized target compound of interest is purified or isolated from a mixture of compounds It is to be noted that Wallis et al. (Chemistry and Biology 1995, Vol. 2(8) p. 543) and Jiang et al. (Chemistry and Biology 1997, Vol. 4(1) p.35) disclose aminoglycoside-aptamer complexes.

Oligonucleotide or oligopeptide aptamers having a selective affinity for the at least one reactive group not to be modified and having no affinity for the at least one reactive site to be modified can be generated by methods known per se in the art. The term aptamer is derived from the Latin 'aptus' meaning "to fit" and is based on the strong binding of oligopeptides or oligonucleotides to specific targets based on structural conformation.. Aptamers are usually created by selecting them from a large random sequence pool. Preferably, an oligonucleotide or oligopeptide aptamer is obtained by screening a library of candidate aptamers and selecting at least one oligonucleotide or oligopeptide displaying the desired characteristics.

In one embodiment, the protective group is an oligonucleotide aptamer, like an RNA or DNA aptamer. Suitable oligonucleotide aptamers are single-stranded RNA or DNA oligonucleotides, that bind with high affinity to specific molecular targets; most aptamers to proteins bind with Kds (equilibrium constant) in the range of 1 pM to 1 nM similar to monoclonal antibodies. The oligonucleotide typically consists of between 5 and 60 nucleic acids, preferably 15-40, nucleotide residues or non-natural nucleotide derivatives. As used herein, the term "nucleotide" encompasses both naturally occurring and (semi)-synthetic nucleotide analogs. For example, non-natural nucleobase-modified nucleotides, representing the latter class, can be also incorporated by polymerases (S. Jäger, M. Famulok, Angew. Chem. Int. Ed. 2004, 43, 3337-3340).

Theoretically, it is possible to select APGs virtually against any molecular target; aptamers have been selected for small molecules, peptides, proteins as well as viruses and bacteria. The APGs are advantageously selected by incubating the target molecule in a large pool of oligonucleotide, the pool size of the oligonucleotide is from 10¹⁰ to 10²⁰. The large pool size of the oligonucleotide ensures the selection and isolation of the specific aptamer. The structural and informational complexity of the oligonucleotide pool and its functional activity is an interesting and active area to develop an algorithm based development of nucleic acid ligands. APGs can distinguish between closely related but non-identical members of a target compound family, or between different functional or conformational states of the same compound. The protocol called systematic evolution of ligands by exponential enrichment (SELEX) is generally used with modification and variations for the selection of specific aptamers. Using this process, it is possible to develop new oligonucleotide APGs in as little as two weeks. Accordingly, in one embodiment a method of the invention involves the use of an oligonucleotide aptamer as protective group, the oligonucleotide aptamer being obtained by a screening process. Starting point of each *in vitro* selection process is typically a synthetic random DNA oligonucleotide library consisting of a multitude of ssDNA fragments with different sequences. This pool of DNA is used directly for the selection of DNA aptamers. For the selection of RNA aptamers, the library has to be transcribed into an RNA library. The SELEX procedure is characterized by the repetition of successive steps consisting of (I) selection (binding, partition, and elution), (II) amplification and (III) conditioning (in vitro transcription or purification of ss DNA). In the first SELEX round the sequence pool and the target molecules are incubated for binding. Non-bound oligonucleotides are removed by several washing steps of the binding complexes. The oligonucleotides that are bound to the target molecule are eluted and subsequently amplified by PCR or RT-PCR. A new enriched pool of selected oligonucleotides is generated by preparation of the relevant ssDNA from the PCR products (DNA SELEX) or by in vitro transcription (RNA SELEX). This selected oligonucleotide pool is then used for the next selection round. In general, 6 to 20 SELEX rounds are needed for the selection of high affinity, target-specific aptamers. The last SELEX round is finished after the amplification step. The enriched aptamer pool is cloned, sequenced and several individual aptamers have to be characterized.

In another embodiment, the APG is an oligopeptide aptamer. As used herein, the term oligopeptide refers to any proteinaceous substance consisting of between about 5 and 120 amino acids, either in the L- or D-configuration. As used herein, the term "amino acid" encompasses both naturally occurring and (semi)-synthetic amino acid analogs. For example, one or more non-natural amino acid analogues can be incorporated into proteins by genetic engineering (C.C.Liu, P:G: Schultz, Ann. Rev. Biochem., 79, 413-44). Typically, a certain minimum size is needed to obtain the desired protective effect. In one embodiment, the oligopeptide aptamer consists of from 8-18 amino acids, preferably 10-13 amino acids.

Methods for selecting an oligopeptide APGs are also known in the art. For example, it involves expressing a library of candidate oligopeptide aptamers in a recombinant host cell, and selecting at least one host cell expressing a desired aptamer and identifying the oligopeptide aptamer. In another embodiment, it comprises the screening of candidate peptides expressed on the cell surface of the host cell. See for example "Decorating microbes: surface display of proteins on Escherichia coli", Bloois E, Winter RT, Kolmar H, Fraaije MW, Trends in Biotechnology, Volume 29, Issue 2, 79-86, 10 December 2010.

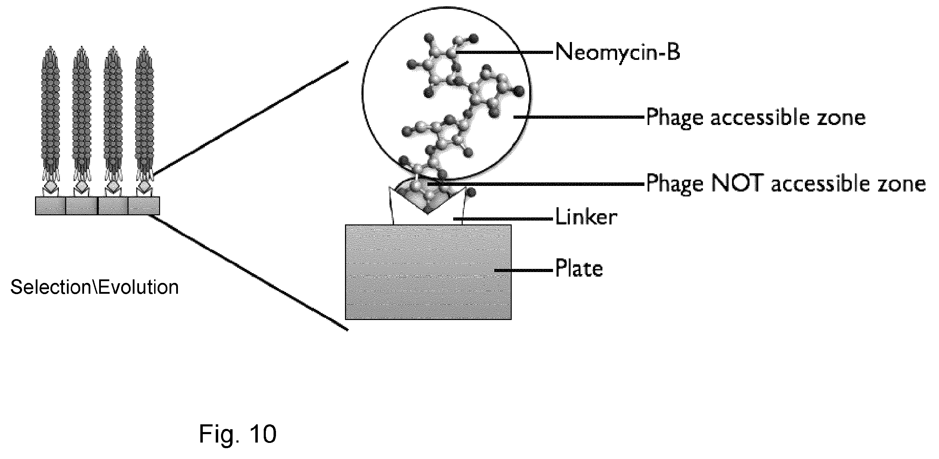

Another suitable method is phage display. Thereby, a library of random peptides is expressed in M13 phages followed by the selection of those phages displaying a peptide that can access and bind to an immobilized target compound. Advantageously, immobilization of the target compound to a solid support is directed in such a manner that the zone which is accessible to the phage comprises the reactive group(s) to be protected against derivatization, whereas the reactive group(s) to be derivatized is/are not accessible to the phages. See also figure 10.

In yet a different approach, a peptide APG is selected by screening the host cell for the ability of the oligopeptide to modulate the biological activity of the target compound. For example, a host cell population is infected with phages expressing a library of random peptides, which is screened for antibiotic resistance to identify oligopeptide aptamers capable of binding to and neutralizing the activity of an antibiotic compound.

The target compound may be a natural product, a synthetic molecule or a semisynthetic derivative of a natural compound. In a preferred embodiment, the target compound is a biologically active compound or a precursor thereof, like a pro-drug. The target compound can be a proteinaceous, like a peptide, or a non-proteinaceous substance, peptides (antibiotics, anti-cancer peptides, genetic diseases, antidiabetics such as exenatide) and

There are many natural products which exhibit multiple functionalities that are applied as therapeutic agent. Prior to the present invention, selective modification of these compounds was difficult or not realizable at all. However, they appear to be very suitable targets for the present APG technology for chemo- and/or regioselective derivatization, thus enabling the facile generation of analogs having improved therapeutic properties.

In one embodiment, the target compound is a macrolides. Macrolides belong to the polyketide class of natural products. The macrolides are a group of drugs (typically antibiotics) whose activity stems from the presence of a macrolide ring, a large macrocyclic lactone ring to which one or more deoxy sugars, usually cladinose and desosamine, may be attached. The lactone rings are usually 14-, 15-, or 16-membered. Macrolides of interest include glycomacrolides (antibiotics, anti-cancer drugs, such as erythromycin), and polyene macrolactons, such as nystatin. In a specific aspect, the target compound is Everolimus (RAD-001), the 40-O-(2-hydroxyethyl) derivative of the macrolide sirolimus. It works similarly to sirolimus as an mTOR (mammalian target of rapamycin) inhibitor and it is currently used as an immunosuppressant to prevent rejection of organ transplants and treatment of renal cell cancer.

In another embodiment, the target compound is a polyphenol. Polyphenols are a heterogeneous class of compounds that include several hydrosoluble antioxidants useful in food preservation and claimed as health promoting agents. Phenolic compounds have attracted special attention due to their health-promoting characteristics. In the past ten years a large number of the studies have been carried out on the effects of phenolic compounds on human health. Many studies have been carried out that strongly support the contribution of polyphenols to the prevention of cardiovascular diseases, cancer, osteoporosis, neurodegenerative diseases, and diabetes mellitus, and suggest a role in the prevention of peptic ulcer. Polyphenols display a number of pharmacological properties in the GIT area, acting as antisecretory, cytoprotective, and antioxidant agents. The antioxidant properties of phenolic compounds have been widely studied, but it has become clear that their mechanisms of action go beyond the modulation of oxidative stress. For example, various polyphenolic compounds have been reported for their anti-ulcerogenic activity with a good level of gastric protection. Preferably, a method of the invention comprises the selective modification of a phenolic compound of pharmaceutical interest, in particular simple phenolic compounds (catechol, eugenol, vanillin, caffeic acid, ferulic acid, and salicin), flavones (apigenin, tangeritin, tangerine, and luteolin), isoflavones (genistein, daidzein, and glycitein), isoflavonoids (rotenone and genistin), flavonols (quercetin, gingerol, kaempferol, myricetin, and rutin), flavanones (hesperetin, naringenin, and eriodictyol), anthocyanidins (cyanidin, delphinidin, malvidin, and petundin), anthocyanins (haematien), coumarins (umbelliferone, aesculetin, and scopoletin), tannins (gallic acids, ellagitannins, catechins, gallitannins, and catechins), lignans (silymarin, matairesinol, pinoresinol, lariciresinol, and secoisolariciresinol), and lignins

Preferred non-proteinaceous substances include saccharides and derivatives thereof. Saccharides are typically divided into four chemical groupings: monosaccharides, disaccharides, oligosaccharides, and polysaccharides. In one aspect, the saccharide derivative is a glycoside. A glycoside is any molecule in which a carbohydrate is bonded through its anomeric carbon to another (non-carbohydrate) group via a glycosidic bond. Glycosides can be linked by an O- (an O-glycoside), N- (a glycosylamine), S-(a thioglycoside), C- (a C-glycoside) or Hal (halogen-glycoside) glycosidic bond. In one embodiment, the invention provides a method for the regioselective transformation of an O-glycoside, S-glycoside, N-glycoside, C-glycoside, or Halogen-glycoside. In a preferred embodiment, the target compound is an aminoglycoside. An aminoglycoside is a molecule or a portion of a molecule composed of amino-modified saccharide.

In a specific aspect, the target compound is an aminoglycoside antibiotic, preferably an antibiotic based on a neamin scaffold, such as neomycin, paromomycin, ribostamycin, kanamycin or streptomycin. As is shown herein below, RNA SPGs allow the convenient functionalization of ring IV of the antibiotic neomycin B with a regioselectivity of up to 98% and conversions of 83% in only one reaction step, whereas conventional synthesis requires more than 20 steps including conventional covalent protection group chemistry accompanied by much lower overall yields. Furthermore, the generality of the concept was demonstrated by employing RNAs with different sequence compositions and for aminoglycosides antibiotics with different functionalities at the pharmacophore, such as neomycin B, paromomycin and kanamycin B. These results demonstrate that SPGs based on oligonucleotides are an indispensable and effective tool for the derivatization of natural products and drugs that can otherwise be only synthesized with demanding efforts and high costs. Herewith, the invention also provides the use of an oligonucleotide or oligopeptide aptamer as a chemo-, regio- and/or stereoselective protective group.

The skilled person will recognize that the concept of the present invention is based on the selection of the appropriate non-covalent APG e.g. by the screening of a library of candidate protective groups. Thus, it is not restricted to the chemical nature of either the target compound, the reactive groups or the derivatization reaction. Useful reactive groups include amines, hydroxyls, hydroxylamines, carboxylic acids, thiols, ketones, aldehydes, enamines, C-C-double and C-C triple bonds. In one embodiment, the target compound comprises at least three, e.g. 3, 4 , 5 or even more, chemically equivalent reactive groups, wherein at least one reactive group is to be derivatized and wherein at least one reactive group is not to be derivatized. In a specific aspect, the target compound comprises at least three chemically equivalent reactive groups, wherein only one reactive group is to be derivatized and wherein the remainder of the reactive groups are not to be derivatized.

As is shown herein below, the APG strategy disclosed in the present invention is compatible with diverse reagents in different derivatization reactions, thus making it a valuable general tool in organic synthesis. For example, the APGs were found to allow the application of a pH ranging from 6.9 to 8.0, different salinities (Na⁺ -ion concentration up to 60 mM and even the presence of a transition metal catalyst. They tolerate both charged and non-charged reagents.

Exemplary derivatization reactions comprise acylation, alkylation, guanidation, oxidation, PEGylation, reductive amination, aza-Michael reaction and urethane formation. For instance, the derivatization reaction comprises oxidation of hydroxyl groups to yield keto- or aldehyde-products, alkylation of amine group via reductive amination, alkylation of amine groups via aza-Michael addition, guanidination of amine groups, acylation of amine groups using activated ester chemistry, synthesis of urea derivatives using isocyanates, or PEGylation by various coupling schemes known in the art. In a specific embodiment, derivatization comprises acylation, thiolation, azide introduction or urea bond formation.

For example, the invention provides for the transformation of neomycin B and paromomycin using isocyanate at a temperature of about 40°C. Using aromatic isocyanate, it was found that the N²(IV) amine group of Neomycin B or Paromomycin is regioselectively transformed. In contrast, the use of aliphatic isocyanates at the same conditions provides the transformation of both amine groups, N⁶(IV) and N²(II) , at the same time. The regioselective transformation of the N⁶(IV) amine group is successful using activated succinimide esters at room temperature.

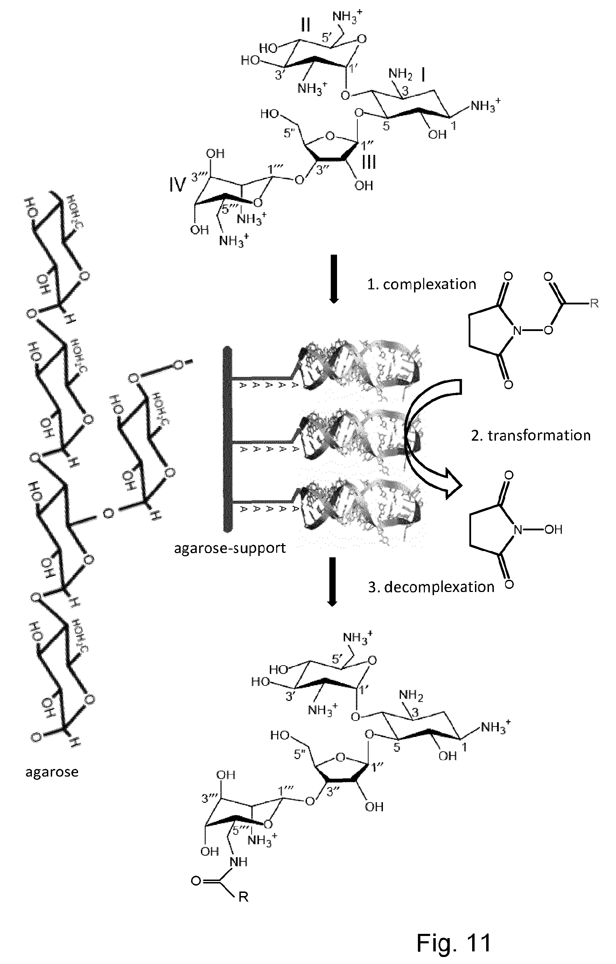

In one embodiment, the host-guest complex is formed while the APG, i.e. the oligopeptide or oligonucleotide aptamer, is in solution. In another embodiment, the host-guest complex is formed while the APG is immobilized. This can be advantageous if the aptamer is to be reused in one or more subsequent derivatization reactions. For example, the aptamer can be covalently bound to a solid support like an agarose column, after which the target compound to be derivatized is allowed to complex to the immobilized aptamer. The derivatization reaction is then performed to selectively modify one or more reactive group(s) while at least one reactive group is protected by the aptamer. Finally, the derivatized target compound is decomplexed from the aptamer after which the cycle can be repeated, e.g. with a different target compound. See also Figure 11.

A specific embodiment of the invention relates to a method for the chemo- and/or regioselective derivatization of an aminoglycoside antibiotic comprising multiple chemically equivalent reactive groups, wherein at least one reactive group is to be derivatized and wherein at least one reactive group is not to be derivatized, the method comprising the steps of
a. contacting the aminoglycoside antibiotic with at least one RNA or oligopeptide aptamer under conditions allowing for the formation of a regioselective host-guest complex, wherein the RNA aptamer has a selective affinity for the at least one reactive group not to be modified; followed and/or accompanied by
b. chemical derivatization of the aminoglycoside antibiotic. Derivatization reactions for providing an aminoglycoside antibiotic derivative, for example derivatives of neomycin, paromomycin, ribostamycin, kanamycin or streptomycin, include acylation, PEGylation and alkylation. In a specific aspect, the invention provides a method for the chemo- and/or regioselective derivatization of neomycin B or paromomycin, wherein at least one reactive amine group is to be derivatized and wherein at least one reactive amine group is not to be derivatized, the method comprising the steps of contacting neomycin B or paromomycin with at least one RNA or oligopeptide aptamer under conditions allowing for the formation of a regioselective host-guest complex, wherein the aptamer has a selective affinity for the at least one reactive amine group not to be modified; followed and/or accompanied by the chemical derivatization, preferably acetylation, of neomycin B or paromomycin.

Preferred RNA aptamers for use in such a method include 5'-GGA CUG GGC GAG AAG UUU AGU CC-3', 5'-CUG CAG UCC GAA AAG GGC CAG-3' and/or 5'-UGU GUA GGG CGA AAA GUU UUA-3'. Preferred oligopeptide aptamers for use in such method include VNRSSDHWNLTT, DYDTLRTVAPTR, NGSLQRSFVISH, HVRIYVDTIEIR, GAMHLPWHMGTL and GAMHPPRHMGPL.

In another specific aspect, the invention provides a method for the chemo- and/or regioselective derivatization of kanamycin A or B, wherein at least one reactive amine group is to be derivatized and wherein at least one reactive amine group is not to be derivatized, the method comprising the steps of contacting kanamycin with at least one RNA or oligopeptide aptamer under conditions allowing for the formation of a regioselective host-guest complex, wherein the aptamer has a selective affinity for the at least one reactive amine group not to be modified; followed and/or accompanied by the chemical derivatization, preferably acetylation, of kanamycin B. In one embodiment, the method allows for selective derivatization of the N⁶ amine of ring II. Preferred RNA aptamers for use in such a method include 5'-GGC ACG AGG UUU AGC UAC ACU CGU GCC-3'.

Also provided is a target compound obtainable by a method of the present invention, or a salt thereof, the compound being characterized in that it is an essentially pure regioisomeric form. The term 'essentially pure' as used herein refers to a regioselectivity of at least about 90%, preferably at least 94%, more preferably at least 97%, most preferably 98, 99 or >99%. Regioselectivity of a derivatization can be determined by methods known in the art, including ¹H-NMR, ¹³C-NMR, Heteronuclear Single Quantum Coherence spectra (HSQC), Attached Proton Test (APT), and combinations thereof.

In one embodiment, there is provided a novel aminoglycoside antibiotic derivative, preferably a derivative based on the neamine scaffold, or a salt thereof. For example, it is a derivative of neomycin, paromomycin, ribostamycin, kanamycin or streptomycin, in an essentially pure regioisomeric form. Preferred derivatives include acylated derivatives, more preferably mono- and diacylated aminoglycoside antibiotics. In a further embodiment, the invention provides a derivatized aminoglycoside antibiotic based on the neamine scaffold. The latter class of compounds are highly attractive candidates for derivatization via a SPG strategy since the design of newly modified antibiotics based on carbohydrates targeting prokaryotic 16S ribosomal RNA (rRNA) has become a powerful tool to achieve higher biological activity against resistant bacteria. However, their chemo- and regioselective build-up cannot be achieved without multi step synthesis including extensive introduction and removal of conventional protective groups and is restricted to a limited number of functionalizable sites. Important examples based on the neamine scaffold are the aminoglycoside antibiotics neomycin B (neoB), paromomycin, and ribostamycin. The possible derivatizations known in the art are limited to enzymatic modifications of the 6-amine group of ring II and chemical transformation of the 5-hydroxy group of ring III, which results in lower antimicrobial activity. In contrast, removal of the positive charge of neomycin B ring IV is tolerated without significantly impairing biological activity and other studies indicate that this structural element of the antibiotic is less involved in interactions with natural RNA molecules, such as rRNA and tRNA. Based on the novel approach using oligonucleotide or peptide aptamers as protective group, the present inventors succeeded in the manufacture of a series of novel aminoglycoside antibiotics. The novel compounds are solely derivatized at one (N² or N⁶) or two amine (N² and N⁶) groups of ring IV. Hence, also provided herein is an aminoglycoside antibiotic derivative, preferably a neomycin,or paromomycin derivative, characterized in that it is derivatized only on the N⁶ (IV)-, N² (IV)- or N² (IV), N⁶ (IV)-amine group(s). The skilled person will understand that numerous types of useful derivatives can be synthesized using the approach disclosed in the present invention.

Provided is an aminoglycoside derivative having the general formula 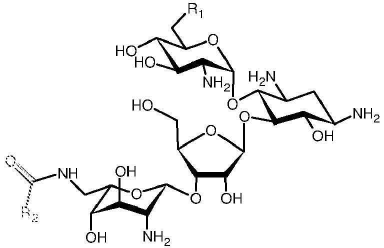 wherein R1 is hydroxyl or amine and wherein R2 is methyl, isopropyl or but-3-inyl.

Also provided is an aminoglycoside derivative having the general formula 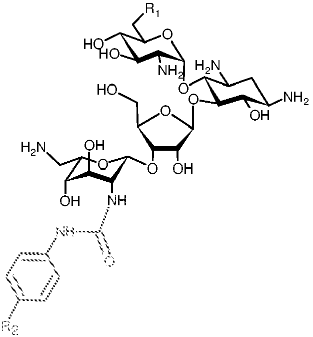 wherein R1 is selected from hydroxyl and amine and wherein R2 is selected from hydrogen, methoxy and N,N-dimethylamine.

Also provided is an aminoglycoside derivative having the general formula 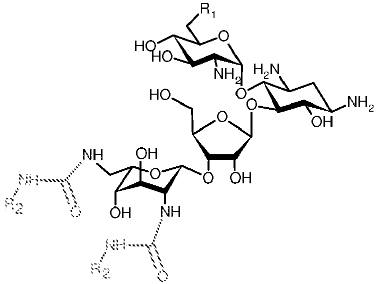 wherein R1 is selected from hydroxyl and amine and wherein R2 is allyl.

Further exemplary novel compounds according to the invention include N⁶(IV) acetyl neomycin B (Formula 1), N⁶(IV) dimethylacetyl neomycin B (Formula 2), and N⁶(IV) pent-3-inoyl neomycin B (Formula 3).

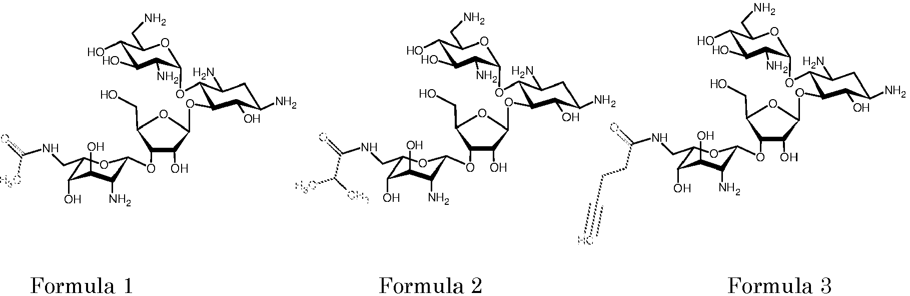

Neomycin B derivatives according to Formula 1, 2 and 3 are not known in the art. Transformation of the amino groups of neomycin B generally only yields derivative mixtures, see Journal of Chromatography (1988), 440, 71-85; FEBS Journal 274 (2007) 6523-6536; Biochimica et Biophysica Acta 1464 (2000) 35-48; Antiviral Research 60 (2003) 181-192. Especially the neomycin B derivative according to Formula 3 exhibits for the first time a terminal acetylene group on the N⁶IV amino group, which can be used for further modification using click-chemistry to provide new and promising neomycin B conjugates with lower bacterial resistance and higher affinity to targets such as the ribosome or HIV-RNA.

Further exemplary compounds include N²(IV) {[(phenyl)amino]carbonyl} amino neomycin B (formula 4), N²(IV) {[(4-methoxyphenyl)amino]carbonyl} aminoneomycin B (formula 5).

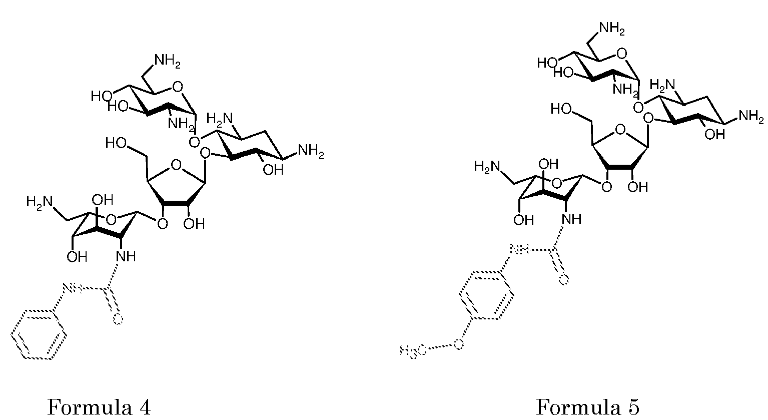

So far, urea- and thiourea- derivatives of neomycin B were synthesized only as a 1:1 mixture of N⁶(IV) and N⁶(II) modified regioisomeres or as fully functionalized urea derivatives reacting five or six amine groups of the antibiotic (Journal of Chromatography B 877 (2009) 1487-1493; Biochimica et Biophysica Acta 1464 (2000) 35-48). Using the novel SPG approach and aromatic isocyanates, the inventors were able to synthesize pure regioisomeres 4 and 5 derivatized on the N²(IV) amino group. Such a functionalization pattern is unprecedented in the literature, presumably because this amino group is not accessible in a regioselective conventional functionalization scheme.

Still further novel neomycin B analogs include N⁶(IV)-y-sulfhydryl-propionyl neomycin B,N⁶-(IV)-azido neomycin B, N⁶-(IV), N²-(IV)-diazido neomycin B, N²(IV)-(propylamino)carbonyl neomycin B, N²(IV)-(isopropylamino)carbonyl neomycin B, N²(IV)-(tert-butylamino)carbonyl neomycin B, N²(IV), N6(IV)-bis-N-(propylamino)carbonyl neomycin B, N²(IV), N⁶(IV)-bis-N-(isopropylamino)carbonyl neomycin B, N²(IV), N⁶(IV)-bis-N-(tert-butylamino)carbonyl neomycin B, or a salt thereof.

It will be understood that the unique, stereospecific derivatization procedure is not limited to neomycin B but that it can be applied to any similar structure. For example, also provided is N²(IV) {[(4-methoxyphenyl)amino]carbonyl}amino paromomycin (Formula 6). Disclosed herein below is the synthesis of N⁶(IV) acetyl paromomycin B

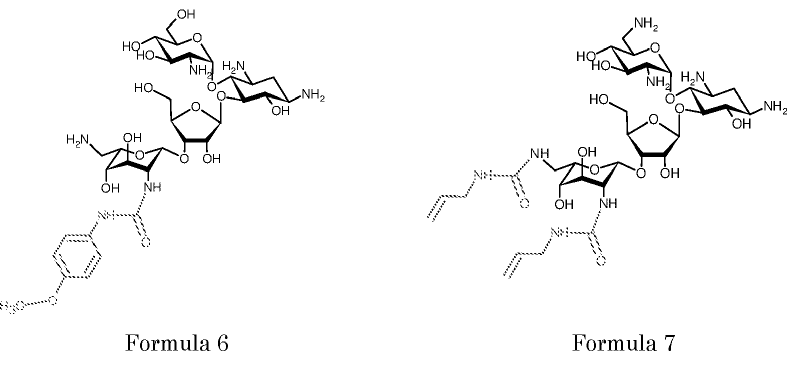

In a still further specific embodiment, there is provided N²(IV),N⁶(IV)-bis{[(allylamino)carbonyl]amino} neomycin B (Formula 7). The novelty of derivative 7 resides among others in the regioselective modification on N⁶(IV) and N²(IV) amine groups at the same time. Preferred functionalities are alkyl groups or (as realized in Formula 7) modifications with terminal olefins for further modification e.g. using metathesis chemistry or Heck-type reactions.

The invention also relates to novel derivatives of kanamycin B wherein the N⁶(II) amine group is derivatized. For example, in one embodiment it provides N⁶(II)-acetyl kanamycin B, N⁶(II)-2-methylpropionyl kanamycin B, N⁶(II)-2-butynyl kanamycin B or N⁶(II)- -sulfhydryl butanoyl kanamycin B or a salt thereof.

Another aspect of the invention relates to a pharmaceutical composition comprising a biologically active derivative, preferably an antibacterial aminoglycoside derivative, of the invention. Provided is a pharmaceutical composition for treatment or prevention of bacterial infection comprising, as an active ingredient a compound which comprises a compound of the invention in a pharmaceutically acceptable form.

For example, provided is a pharmaceutical composition comprising at least one aminoglycoside antibiotic selected from the group consisting of N⁶(IV) acetyl neomycin B, N⁶(IV) dimethylacetyl neomycin B, and N⁶(IV) pent-3-inoyl neomycin B, N²(IV) {[phenyl)amino]carbonyl}amino neomycin B, N²(IV) {[(4-methoxyphenyl)amino]carbonyl} aminoneomycin B, N²(IV) {[(4-methoxyphenyl)amino]carbonyl}amino paromomycin and N²(IV), N⁶(IV)-bis{[(allylamino)carbonyl]amino} neomycin B, N⁶(IV)-y-sulfhydryl-propionyl neomycin B, N²(IV)-(propylamino)carbonyl neomycin B, N²(IV)-(isopropylamino)carbonyl neomycin B, N²(IV)-(tert-butylamino)carbonyl neomycin B, N²(IV), N6(IV)-bis-N-(propylamino)carbonyl neomycin B, N²(IV), N⁶(IV)-bis-N-(isopropylamino)carbonyl neomycin B, N²(IV) , N⁶(IV)-bis-N-(tert-butylamino)carbonyl neomycin B, N⁶(II)-acetyl kanamycin B, N⁶(II)-2-methylpropionyl kanamycin B, N⁶(II)-2-butynyl kanamycin B or N⁶(II)- -sulfhydryl butanoyl kanamycin B, or a pharmaceutically acceptable salt thereof, together with a pharmaceutically active diluent or excipient.

Aminoglycoside antibiotics have been commonly used as a medical treatment against infectious diseases for over 60 years, although the prevalence of aminoglycoside resistant bacteria has significantly reduced their effectiveness. Aminoglycosides have two or more amino sugars bound to an aminocyclitol ring through glycosidic bonds. Naturally occurring aminoglycosides (produced by Actinomycetes) are widely used as antibiotics against bacterial infections of animals and humans. These include the well-known antibiotics kanamycin, streptomycin and neomycin. Aminoglycoside antibiotics are believed to act on the bacterial protein synthesis machinery, leading to the formation of defective cell proteins.

The aminoglyoside derivative of the invention, preferably an N-substituted aminoglycoside derivative, possesses utility as antibiotic or as intermediate in the preparation of aminoglycoside antibiotics. Hence, also provided is an aminoglycoside derivative according to the invention for use as a medicament, for instance in a method of treatment of a bacterial infection. In one embodiment, a method of treating or preventing a bacterial infection comprises administering a bacteriostatic or bacteriocidal amount of the aminoglycoside derivative to a mammalian host in need thereof. The term "administering" is defined herein to describe the act of providing, exposing, treating, or in any way physically supplying or applying a chemical or compound to any living organism or inanimate object associated with a living organism, where said organism will actually or potentially benefit for exposure, treatment, supplying or applying of said chemical or compound. An aminoglycoside derivative according to the invention is suitably used in a method of treatment of a bacterial infection, preferably an infection with Methicillin-resistant *Staphylococcus aureus* (MRSA) or vancomycin-resistant enterococci (VRE).

The exact route of administration, dose, or frequency of administration would be readily determined by those skilled in the art and is dependant on the age, weight, general physical condition, or other clinical symptoms specific to the patient to be treated. For example, in medicine, a topical medication is applied to body surfaces such as the skin or mucous membranes, for example throat, eyes and ears. The term "topical" is defined herein as pertaining to the surface of a body part, surface part of a plant, or surface of an inanimate object or composition, such as soil.

The skilled person would know how to formulate the compounds used to practice the method claimed in this invention into appropriate pharmaceutical dosage forms. Examples of the dosage forms include oral formulations, such as tablets or capsules, or parenteral formulations, such as sterile solutions. When the compound used to practice the method claimed in this invention are administered orally, an effective amount is from about 1 to 100 mg per kg per day. A typical unit dose for a 70 kg human would be from about 50 mg to 1000 mg, preferably 200 mg to 1000 mg taken one to four times per day. Either solid or fluid dosage forms can be prepared for oral administration.

Solid compositions are prepared by mixing the compounds used to practice the method claimed in this invention with conventional ingredients such as talc, magnesium stearate, dicalcium phosphate, magnesium aluminum silicate, calcium sulfate, starch, lactose, acacia, methyl cellulose, or functionally similar pharmaceutical diluents and carriers. Capsules can be prepared by mixing the compounds used to practice the method claimed in this invention with an inert pharmaceutical diluent and placing the mixture into an appropriately sized hard gelatin capsule. Soft gelatin capsules can be prepared by machine encapsulation of a slurry of the compounds used to practice the method claimed in this invention with an acceptable inert oil such as vegetable oil or light liquid petrolatum. Syrups may be prepared by dissolving the compounds used to practice the method claimed in this invention in an aqueous vehicle and adding sugar, aromatic flavoring agents and preservatives. Elixirs can be prepared using a hydroalcoholic vehicle such as ethanol, suitable sweeteners such as sugar or saccharin and an aromatic flavoring agent. Suspensions are suitably prepared with an aqueous vehicle and a suspending agent such as acacia, tragacanth, or methyl cellulose.

When the compound used to practice the method claimed in this invention are administered parenterally, it can be given by injection or by intravenous infusion. The effective dosage will depend on the active compound and/or subject to be treated. Typically, an effective amount is from about 1 to 100 mg per kg per day. Parenteral solutions are usually prepared by dissolving the compound in water and filter sterilizing the solution before placing in a suitable sealable vial or ampule. Parenteral suspensions can be prepared in substantially the same way except a sterile suspension vehicle is used and the compounds used to practice the method claimed in this invention are sterilized with ethylene oxide or suitable gas before it is suspended in the vehicle.

The aminoglycoside antibiotic may be given topically in sulphate form, for example in an irrigation solution or topical cream.

One use is in presurgical patient preparation, to eliminate infectious organisms and bring down on the risk of infection. For intestinal surgery, surgeons may use e.g. a neomycin B derivative of the invention in irrigation fluid to keep the surgical wound clean and wash out any infectious organisms and debris. This will cut down on the risks during healing, keeping the patient's gut as healthy as possible. It is also available to irrigate the eyes and nose, or to treat skin infections. Patients may receive this drug in a hospital environment or in a prescription to use at home.

The novel antibiotic may be paired with at least a second useful drug compound, for example a steroid, for the treatment of some conditions, sometimes in a compounded formula featuring both medications. In one example, compounds of the present invention can be formulated independently or together in a pharmaceutically acceptable form and administered to a patient in need thereof. In a more specific example, the pharmaceutically acceptable form could be in a spray or topical solution. In yet more specificity, the spray or topical solution could be used to treat infections with MRSA or VRE.

In an alternative to the more specific example above, the pharmaceutically acceptable form could be such that direct injection or intravenous administration of the compound or compounds of the present invention is the desired route for administering the compound or compounds of the present invention. In yet more specificity, the compound or compounds of the present invention could be used to treat infections caused by Gram-positive or Gram- negative infections.

One skilled in the art would certainly instantly envision using the compound or compounds of the present invention in combination with each other or in combination with other antimicrobial treatments. One skilled in the art would also certainly envision combining topical and intravenous or direct injection administration techniques to achieve synergistic effects. Other combinations of antibiotic compounds and routes of administration, both current in the art and yet to be developed, can or will be envisioned by those skilled in the art. Combined medications can be useful for making sure patients take all their medications and get them in the right dosage. With eye care, for example, intolerance of eye drops can make it difficult for patients to adhere to a dosing regimen, and a combination solution will reduce the risks of this problem by allowing the patient to take both medications in a single eye drop or eyewash.

Formulations of neomycin B derivatives in sprays and creams are provided to treat cuts, scrapes, and other skin infections. The antibiotic can be prophylactic in nature, preventing the onset of infection by making the environment hostile to bacteria. It can also be suitable for treating some kinds of infections, depending on the organism causing the problem and the patient's responsiveness to the drug. In one embodiment, an aminoglycosyl derivative of the invention, for instance a paramomocyin derivative like N²(IV) {[(4-methoxyphenyl)amino]carbonyl}amino paromomycin is used in a method for destroying or suppressing the growth of amoebas.

### LEGEND TO THE FIGURES

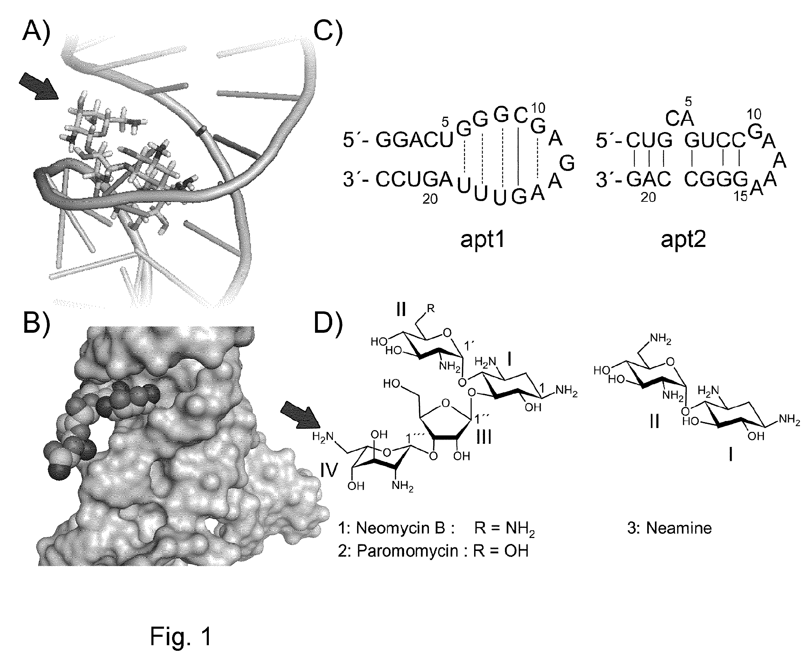

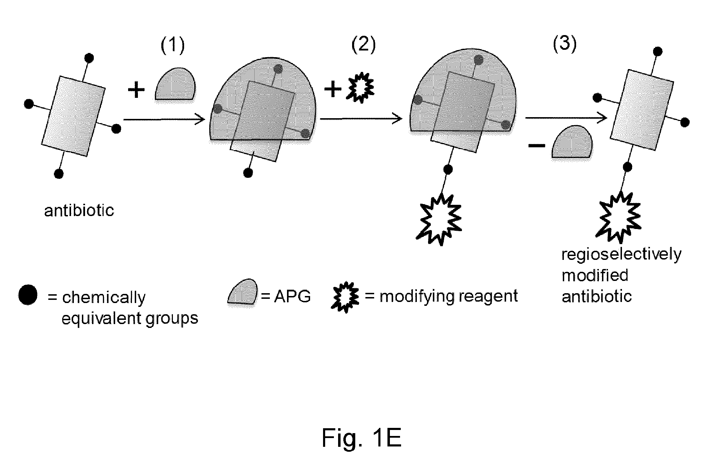

Figure 1. : A) neomycin B RNA aptamer complex; B) model of neomycin B - tRNA interaction based on the basis of the crystallographic coordinates from Mikkelsen²⁷; C) sequence of RNA aptamers apt1 and apt2; D) structure of representative aminoglycoside antibiotics. The arrow indicates the free amine group of neomycin B for derivatization; The arrow indicates ring IV, which is directed outwards towards the solvents in 10 mM phosphate buffer. E) Concept of chemo- and regioselective modification of a complex molecule applying APG technology. (1) non-covalent protection of target antibiotic with APG; (2) selective modification of unprotected functional group(s); (3) deprotection of the new derivative.
Figure 2. : ESI-Mass spectra of reaction mixture on neoB 1 with activated ester 4a in presence of RNA (panel A) and without RNA (panel B). 1H-NMR (500 MHz, D2O) of monoacetylated neomycin B in presence of the aptamer apt1 (panel C) and without apt1 (panel D). (A) and (B) S = neomycin B, I-VI = degree of acetylation of neoB
Figure 3. : regioselective transformation of N6(IV) amine group of aminoglycosides.

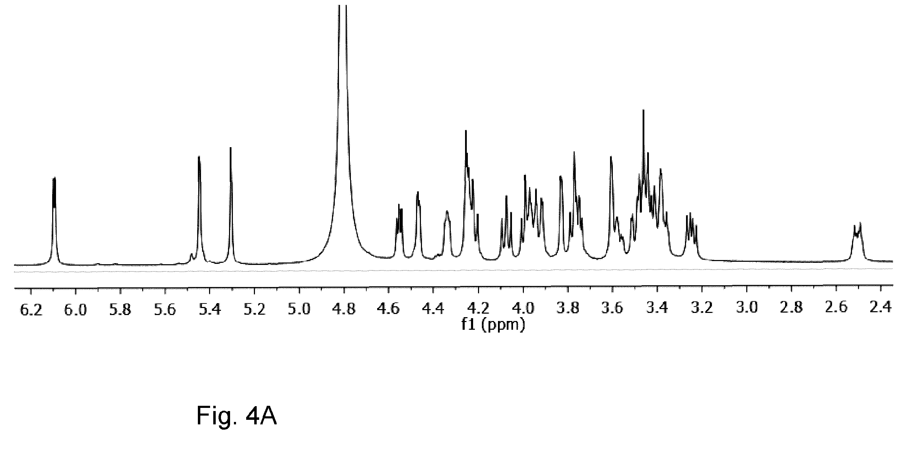

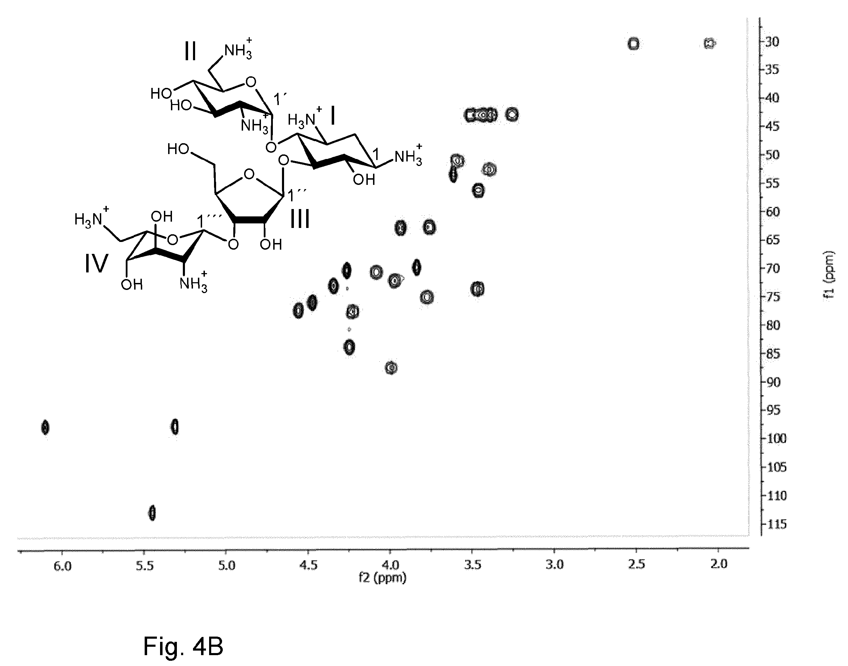

Figure 4: Panel (A) ¹H-NMR spectrum (500 MHz, D₂O) of neomycin B x 6 HFBA 1.
Panel (B) HSQC spectrum (500 MHz, D₂O) of neomycin B x 6 HFBA 1.

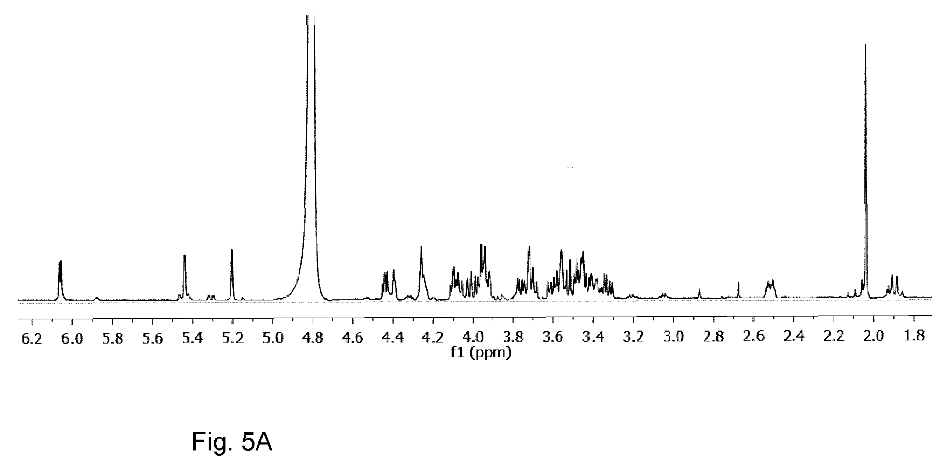

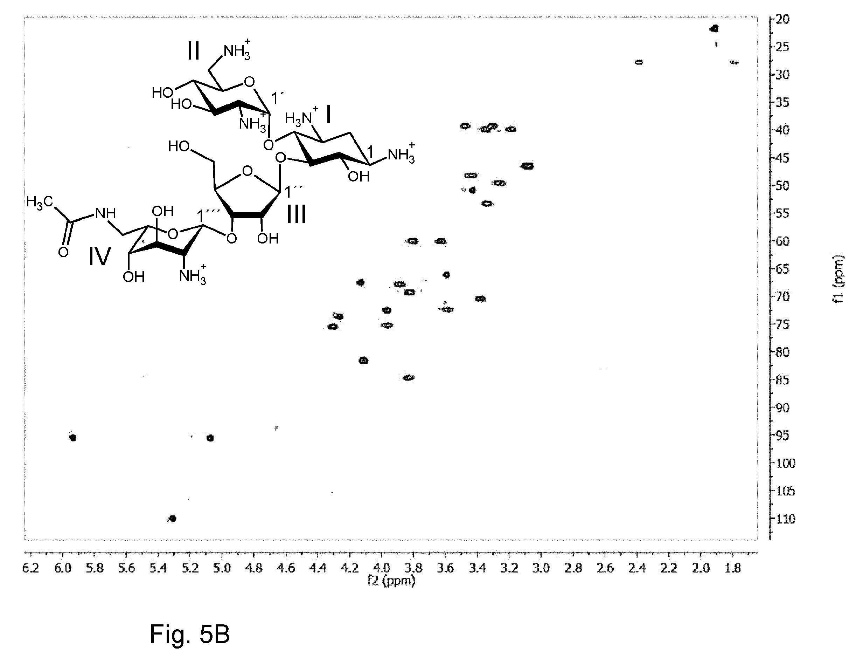

Figure 5: Panel (A) ¹H-NMR spectrum (500 MHz, D₂O) of N⁶(IV)-acetyl neomycin B x 5 HFBA 5a. (panel B) HSQC spectrum (500 MHz, D₂O) of N⁶(IV)-acetyl neomycin B x 5 HFBA 5a.

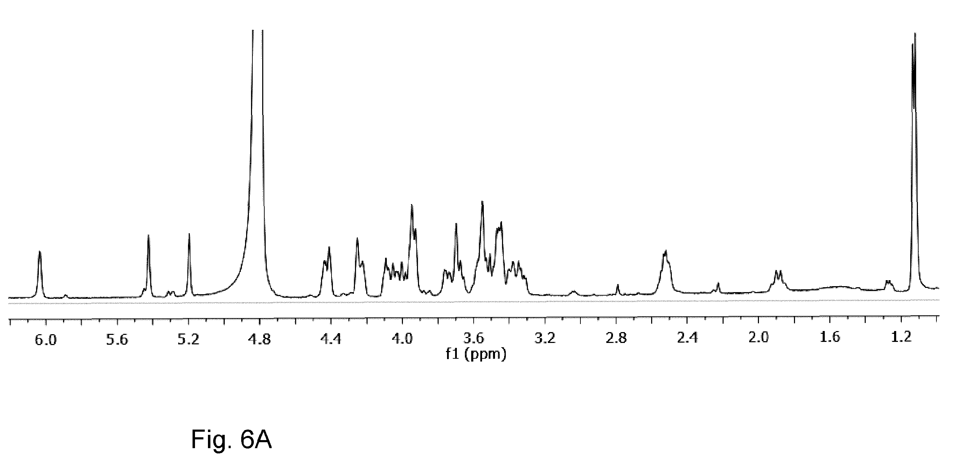

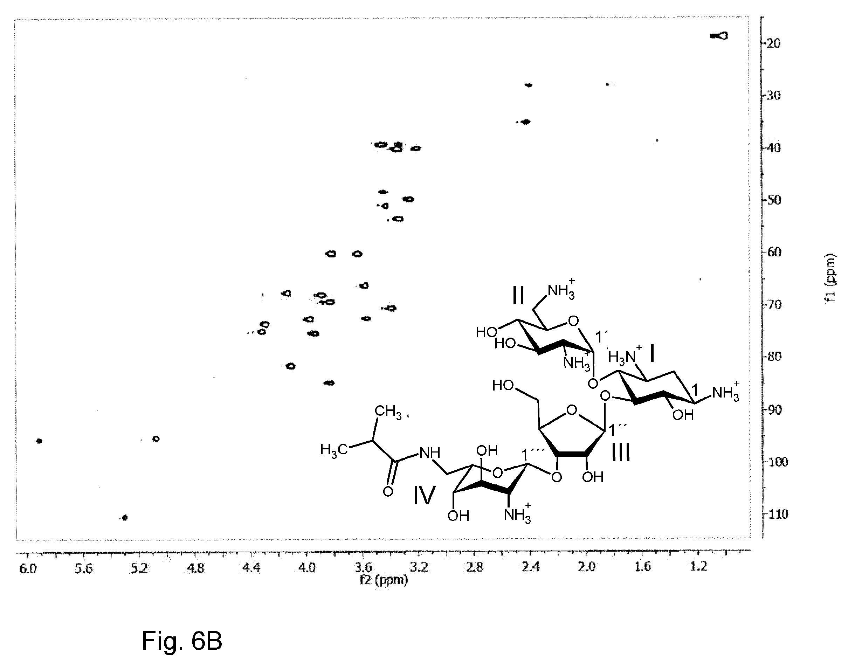

Figure 6: Panel (A) ¹H-NMR (500 MHz, D₂O) spectrum of N⁶(IV)-2,2-dimethylacetyl neomycin B x 5 HFBA 6. Panel (B) HSQC (500 MHz, D₂O) spectrum of N⁶(IV)-2,2-dimethylacetyl neomycin B x 5 HFBA 6.

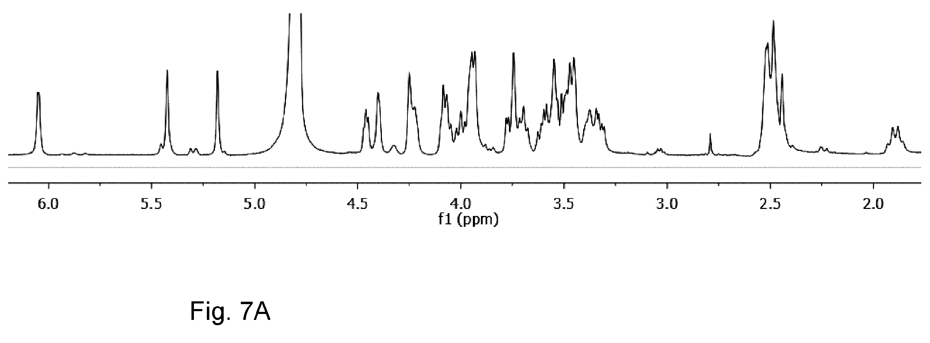

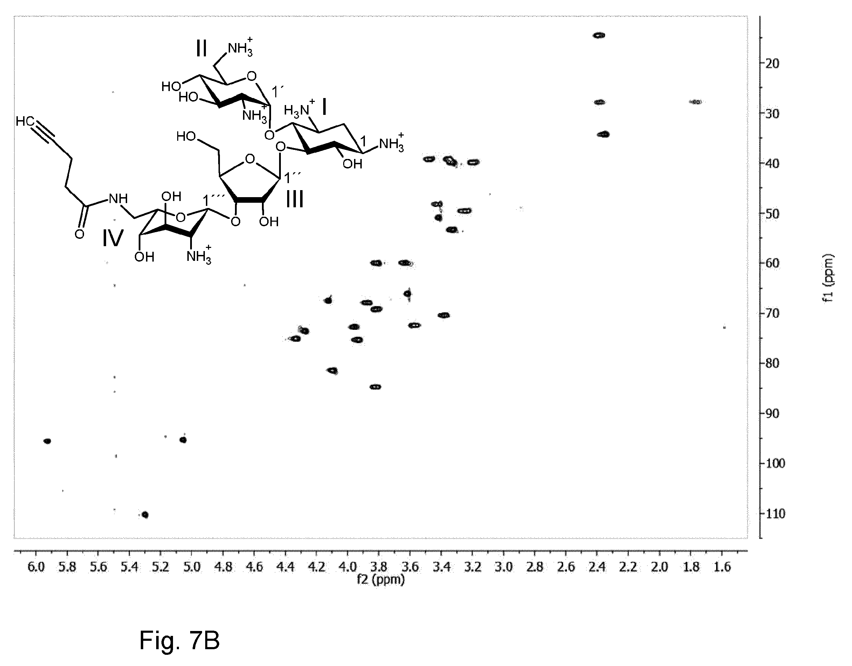

Figure 7: Panel (A) ¹H-NMR (500 MHz, D₂O) spectrum of N⁶(IV)-prop-3-inoyl neomycin B x 5 HFBA 7. Panel (B) HSQC (500 MHz, D₂O) spectrum of N⁶(IV)-prop-3-inoyl neomycin B x 5 HFBA 7.

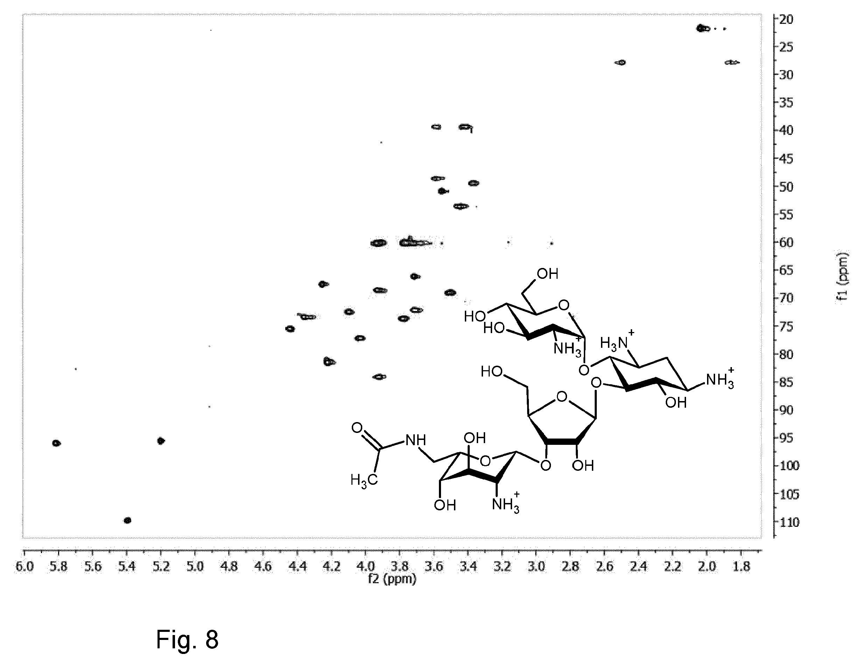

Figure 8: Panel (A) HSQC (500 MHz, D₂O) spectrum of N⁶(IV)-acetyl paromomycin x 4 HFBA 8.

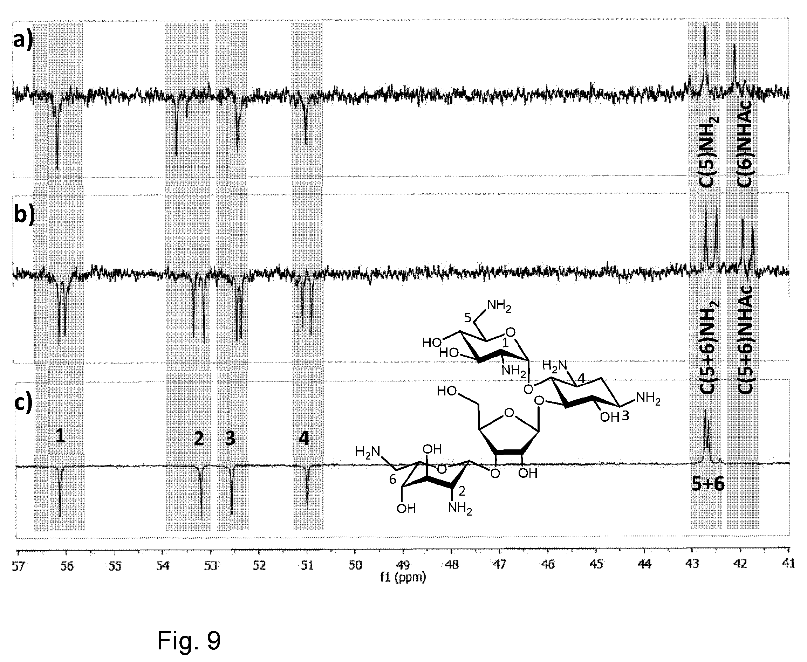

Figure 9: Attached Proton Tests (APT) of monoacetylated neomycin B after transformation in presence of RNA (panel A) and in absence of RNA (panel B). APT of neomycin B (panel C). It proves the regioselective transformation of the N⁶(IV) amine group of the neomycin B to 5a, while the spectrum in panel B shows the presence of two monoacetylated neomycin B derivatives, the N(IV)⁶ acetyl neomycin B 5a and the N(II)⁶ acetyl neomycin B 5b.
Figure 10: Schematic representation of the use of phage display technology to select oligopeptide aptamers. Immobilization of a target compound (e.g. antibiotic) to a solid support is directed in such a manner that the zone which is accessible to the phage comprises the reactive group(s) to be protected against derivatization, whereas the reactive group(s) to be derivatized is/are not accessible to the phages. See also Example 2.
Figure 11: Schematic illustration of the regioselective modification of the neamine antibiotic Neomycin B using immobilized aptameric protective groups (APGs) on solid support (agarose): 1. Complexation of antibiotic with immobilized APG; 2. Acylation using N-hydroxysuccinimide ester; 3. Elution of modified antibiotic.

### EXPERIMENTAL SECTION

### EXAMPLE 1

This Example demonstrates the power of the novel SPG strategy for the highly regioselective modification of aminoglycoside antibiotics to obtain new and biologically active saccharide derivatives within a single reaction step. It is proven for the first time that non-covalent protective groups can be generated with the help of a well established *in vitro* evolution process and do not require the tailored design and synthesis of ligands to protect individual target molecules for highly selective chemical transformations.

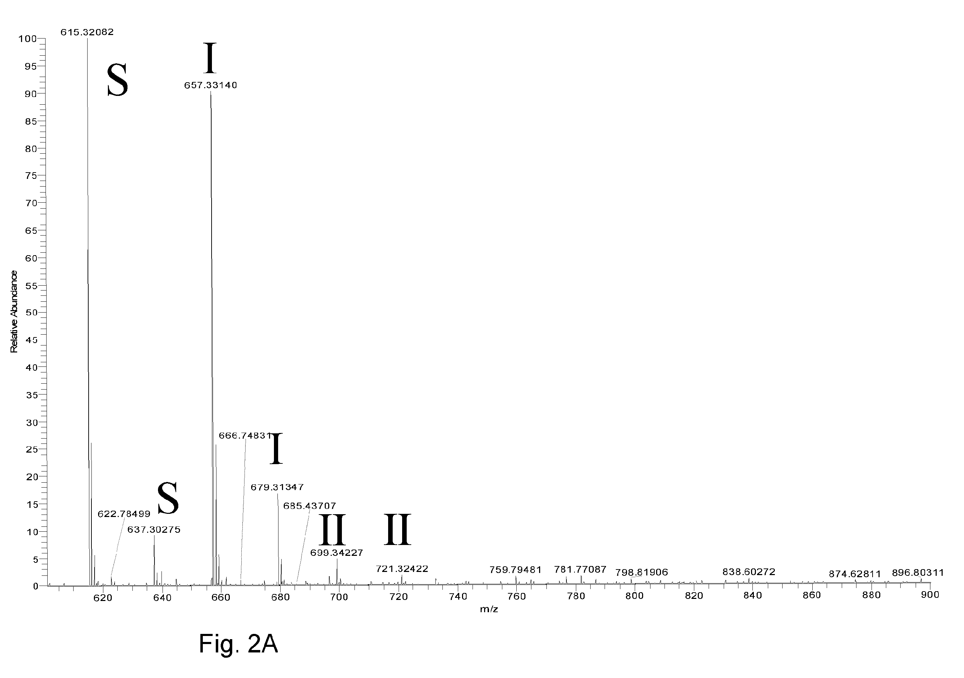

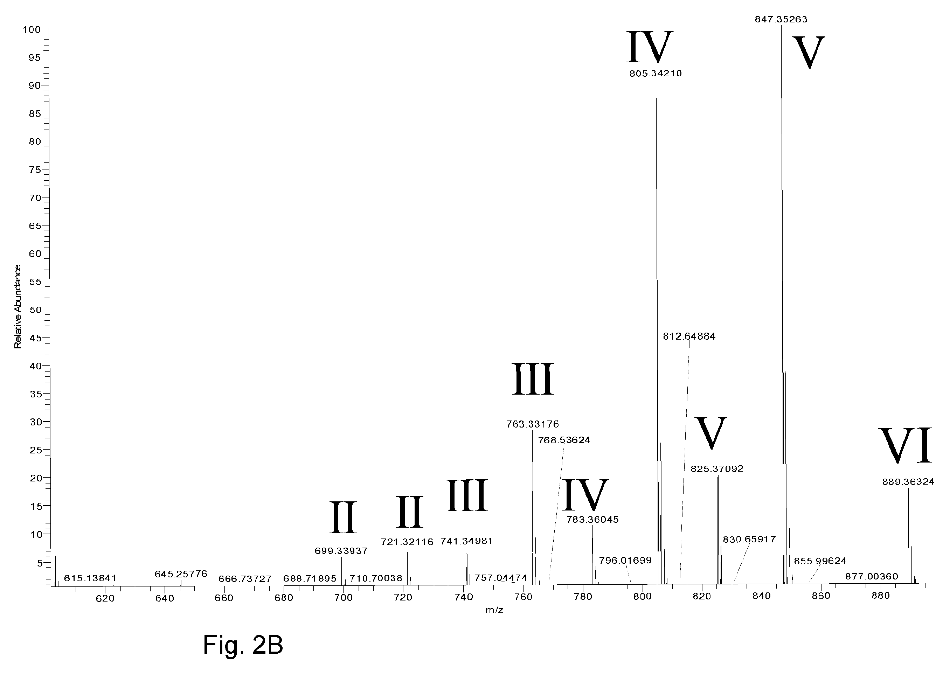

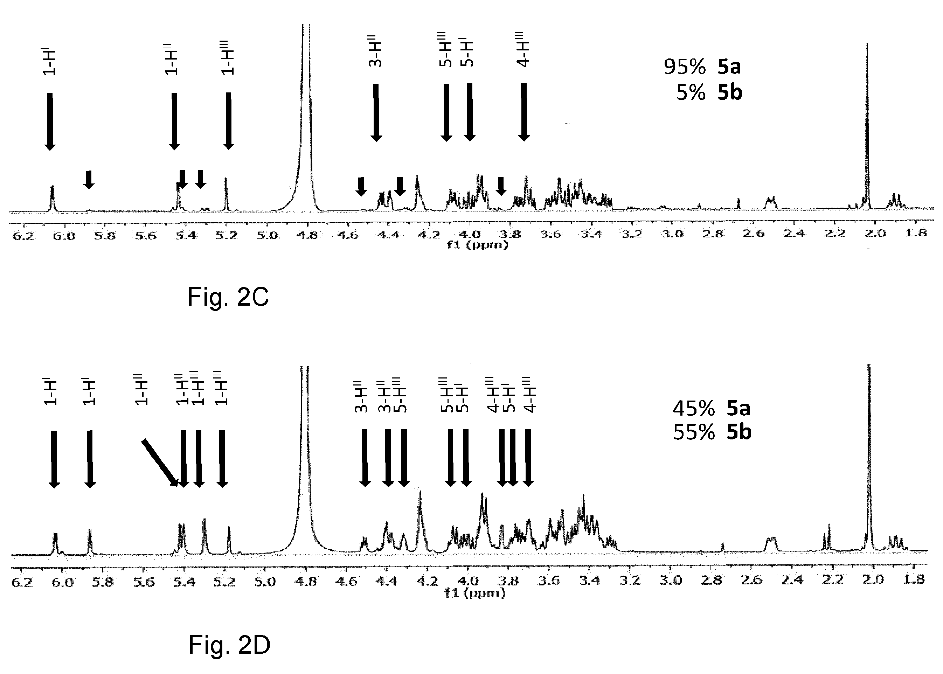

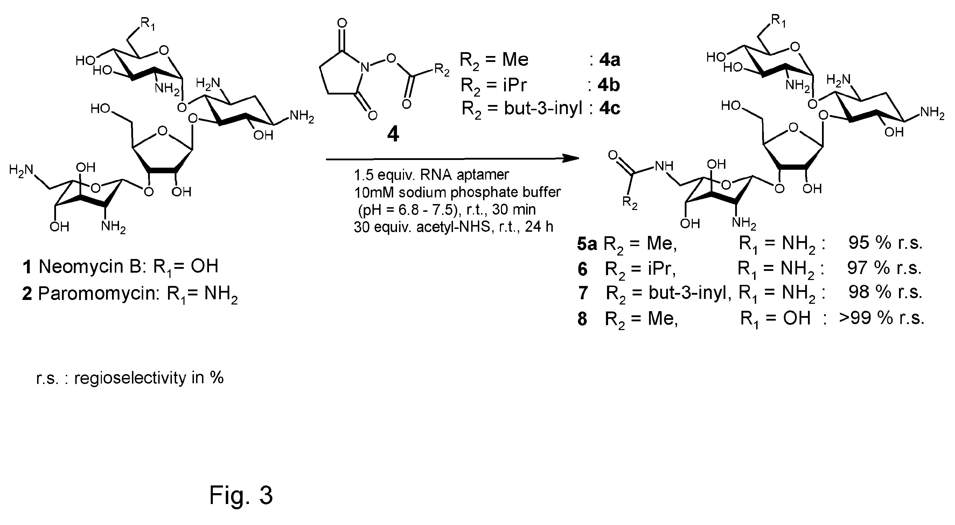

For the straightforward proof of concept that RNA can act as a SPG, a well characterized host-guest complex of an 23mer RNA aptamer (sequence: 5'-GGA CUG GGC GAG AAG UUU AGU CC-3', **apt1)** and neoB was chosen. X-ray crystallography and nuclear magnetic resonance (NMR) measurements revealed that one of the six amine groups, i.e., the 6-amine group of ring IV, is not involved in complex formation and extends into the solvent (Fig. 1A)²⁹. Reaction of the N-acetoxy succinimide ester (10 equivalents) with the **apt1** protected neoB resulted only in the formation of mono- and diacetylated neoB molecules according to mass spectrometric analysis (Fig. 2A). In stark contrast, the unprotected transformation of neoB under the same conditions yielded di-, tri-, tetra-, penta and hexaacetylated products of the antibiotic (Fig. 2B). These experiments clearly indicated the ability of **apt1** to inhibit the reactivity of several amine groups present within neoB. To obtain further insights into the regioselectivity of the transformations at the neoB scaffold reaction conditions were optimized to yield monoacylated neoB for the protected (Fig. 3 and table 1) and unprotected transformations. RNA-neoB complexes were reacted with 30 equivalents of the activated ester **4a** while the unprotected neoB was incubated with only one equivalent of the succinimidyl ester. After purification by high performance liquid chromatography (HPLC) the monoacyl-neoB products were characterized and regioselectivities were determined by ¹H-NMR spectroscopy. It turned out that the RNA-protected neoB was acylated at the 6-amine group of ring IV with an extremely high regioselectivity of 95% to form **5a** (Fig. 2C) while the unprotected neoB was converted to N⁶(IV)-acetyl neoB **5a** and the N⁶(II)-acetyl neoB **5b** in a ratio of 9:11 (Fig. 2D). These results were confirmed by Heteronuclear Single Quantum Coherence spectra (HSQC) and Attached Proton Test (APT) measurements. To test whether larger residues at the acyl group are tolerated by **apt1,** neoB was functionalized by employing the succinimidyl esters of isobutyric acid **4b** and 4-pentynoic acid **4c** (Fig. 3 and Tab. 1). Again, remarkable regioselectivities of 97% and 98% were achieved for **6** and 7, respectively, even exceeding the values of the acetylation reaction. It is important to mention that **apt1** during preparation of **6** and **7** even tolerated the addition of the organic solvent dimethylformamide (DMF), which allowed easy solubilization of the hydrophobic activated esters **4b** and **4c.** Besides the high regioselectivities found for all the acylation reactions, the conversions for obtaining **5a, 6** and **7** were very high with values of 76%, 71% and 83%, respectively.

To demonstrate the generality of the concept regarding nucleotide aptamers, an even shorter 21mer aptamer (sequence: 5'-CUG CAG UCC GAA AAG GGC CAG-3', **apt2)** was employed as SPG for neoB (tab. 1). This oligonucleotide was obtained in the same SELEX experiment as **apt1¹⁰.** Again, high regioselectivities were obtained for **5a, 6** and **7** (94%, 89% and 96%, respectively). The conversions for the reactions were slightly lower than for **apt1,** reaching 63%, 59% and 67%, respectively.

To show that the SPGs mentioned above are also valuable for the generation of other drug derivatives with a similar pharmacophore, the related antibiotic paromomycin **2** was subjected to an acylation reaction employing **apt1** (Fig. 1 and Table 1). It was found that **2** was exclusively transformed into the regioisomer N⁶(IV)-acetyl paromomycin **8.**

After the successful synthesis of novel neomycin B derivatives the compounds **5a** and **6** were investigated regarding their antimicrobial activities against *E. coli* ATCC 25922, which is a standard strain to evaluate the efficiency of antibiotics¹. Two methods, the Kirby-Bauer Disk Test and the determination of the Minimal Inhibitory Concentration (MIC), were employed for that purpose (Table 2). It turned out that, despite of the removal of the positive charge of ring IV by acylation, the aminoglycoside derivatives **5a and 6** were still highly active. As shown in Table 2 the activity of **5a** exhibiting the acetyl residue is slightly lower than neoB **1** with MIC-values of 6.3 and 3.1 µM, respectively, while the derivative **6** modified with the more hydrophobic isobutyrate residue shows the same biological activity as paromomycin **2.** These results confirm the finding that the 6-amine group of neomycin B ring IV is well suited for functionalization of the antibiotic and at the same time the high affinity of the neamine core **3** (ring I and II, Fig. 1D) to the prokaryotic ribosomal RNA is maintained¹.

In conclusion, the effective use of short RNA sequences as non-covalent SPGs was demonstrated for the highly chemo- and regioselective derivatization of complex molecules bearing several functional groups with similar reactivity. It should be emphasized that the generation of such protective groups based on oligonucleotides relies on a well-established in vitro evolution process and binders for a large variety of target molecules bearing different structural features can be evolved. In regard to the previously reported macrocyclic hosts^{4,5}, the current limitations of SPG strategies, i.e., being only effective for molecules with a simple structure and the need for complicated design and synthesis of the host, were overcome. RNA SPGs allow the convenient functionalization of ring IV of the antibiotic neoB **1** with a regioselectivity of up to 98% and conversions of 83% in only one reaction step whereas conventional synthesis requires more than 20 steps including conventional covalent protection group chemistry¹ accompanied by much lower overall yields. Furthermore, the generality of the concept was demonstrated by employing RNAs with different sequence compositions and for aminoglycosides antibiotics with different functionalities at the pharmacophore, such as neomycin B **1** and paromomycin **2**. These results suggest that SPGs based on oligonucleotides will become an indispensable and effective tool for the derivatization of natural products and drugs that can otherwise be only synthesized with demanding efforts and high costs.

**Table 1: Regioselectivity of transformation dependent on size of activated ester**

| antibiotic | aptamer | R₂ | Conv. ^ (%) | r.s.*(%) |
|---|---|---|---|---|
| 5a | apt1 | Me | 76 | 95** |
| 5a | apt2 | Me | 63 | 94 |
| 6^{†} | apt1 | *i*-Pr | 71 | 97** |
| 6^{†} | apt2 | *i*-Pr | 59 | 89 |
| 7^{†} | apt1 | 3-butinyl | 83 | 98** |
| 7^{†} | apt2 | 3-butinyl | 67 | 96 |
| 8 | apt1 | Me | 60 | >99 |

| | | | | |
|---|---|---|---|---|
| All reactions were carried out using the general procedure (Supplementary Information) * regioselectivity (r.s.) of monoacylated neomycin B determined by ¹H-NMR, HSQC and APT. ** regioselectivity average of three runs ^ maximal observed conversion of neomycin B to N⁶ acyl neomycin B as isomere mixture † reaction were carried out in a mixture of 10 mM sodium phosphate buffer pH 6.8 and 6.7% DMF | | | | |

**Table 2. : Antimicrobial Activity of Aminoglycosides against E. coli ATCC 25922**

| Antibiotic | Amount (nmol) | Diameter^{a} (mm) | MIC^{b} (µM) |
|---|---|---|---|
| 1* | 17.5 | 16.8 | 3.1 |
| 1^ | 17.5 | 16.1 | 3.1 |
| 2^ | 17.5 | 13.2 | 12.5 |
| 5a | 17.5 | 13.9 | 6.3 |
| 6 | 17.5 | 11.5 | 12.5 |

| | | | |
|---|---|---|---|
| * All compounds were used as HFBA salts, except for neomycin B sulphate 1* (Sigma Aldrich) ^ antibiotic x 6 HFBA, purified by HPLC using the same conditions of the neomycin B derivatives **5d and 6** (see Methods Summary). All compounds were used as HFBA salts, expect of neomycin B sulphate **1*** (Sigma Aldrich) ^{a} The zones of inhibition as determined by Kirby-Bauer disk method are given. For all compounds, the molar amount was kept constant at 17.5 mmol. ^{b} The minimum inhibitory concentrations are given in µM. | | | |

### General procedure for the synthesis and testing of antibiotic derivates 5a, 6, 7 and 8.

### Materials

All chemicals and reagents were purchased from commercial suppliers and used without further purification, unless otherwise noted. Neomycin B trisulfate x hydrate (VETRANAL®), paromomycin sulfate salt (98%), *N,N*-dimethylformamide (DMF, 99%), *N*-hydroxysuccinimide (NHS, 98%), trifluoroacetic anhydride (99%), dichloromethane (DCM, 99.5%), tetrahydrofurane (THF, 99.9%), pyridine (99%), 4-pentynoic acid (95%), acetic acid (99%), isobutyric acid (99%) and toluene (99.8%) were purchased from Sigma Aldrich and used as received. For HPLC purification heptafluorobutyric acid (HFBA) (Fluka, puriss. p.a., for ion chromatography), acetone (Sigma-Aldrich, HPLC grade) were used. Ultrapure water (specific resistance > 18.4 MΩ cm) was obtained by Milli-Q water purification system (Sartorius®). RNA aptamers (82 - 91 % of purity) were purchased from *BioSpring* (Frankfurt am Main, Germany). All used *N*-hydroxysuccinimide ester **(4a-c)** were prepared according to standard literature procedures^{1,2}. For the regioselective transformation Milli-Q water was treated with diethylpyrocarbonate (DEPC) and sterilized using an autoclave (121 °C, 20 min).

### General procedure for regioselective transformation of aminoglycosides

The general procedure for regioselective transformation of aminoglycosides is schematically outlined in Figure 3.

816 µL of a 6.1 mM RNA aptamer solution in 10 mM sodium phosphate buffer (pH 6.8) were heated to 85 °C for 10 min and afterwards stored for 15 min at room temperature. 684 µL of a 4.8 mM solution of the antibiotic (3.28 µmol) in 10 mM sodium phosphate buffer (pH 7.5) were added and the mixture was allowed to stand for 30 min at room temperature. 30 equiv. activated ester (98.4 µmol) dissolved in 1.5 mL sodium phosphate buffer (pH 7.5) (for activated ester **4a**) or in 106 µl DMF (for activated esters **4b** and **4c)** were added and the reaction mixture was allowed to react for 24 hours at room temperature. After addition of 126 µL of a 7 wt. % ethylamine water solution and further incubation for 30 min at room temperature the crude mixture was heated to 95 °C for 10 min. To the hot solution 3 mL of a 53 mM aqueous solution of didodecyldimethylammonium bromide (DDDMABr) were added to precipitate the RNA. After incubation for 15 min at room temperature and centrifugation for 30 min at 6 °C (16.1 u/s) the supernatant was freeze dried and dissolved in 400 µL water. Each 30 µL fraction was purified by HPLC using a Waters Spherisorb ODS-2C₁₈ analytic column (water/acetone 6 : 5 containing 11.5 mM HFBA) and a flow rate of 1 ml/min at 40°C to afford the antibiotic derivatives **5a, 6, 7** and **8.**

### Analytical Data

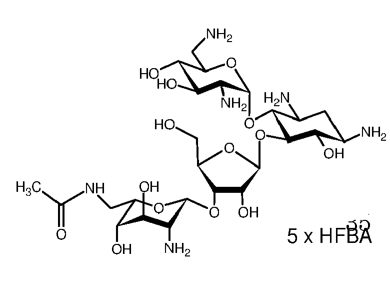

**N⁶(VI)-acetyl neomycin B x 5 HFBA (5a).** The title compound was prepared according to the general procedure described above. Derivative **5a** was obtained as a white solid. For the measurement of regioselectivity and the characterization of the compound ¹H-NMR, HSQC as well as APT spectra were recorded and electrospray ionization (ESI)-MS was employed. The yield was determined by HPLC: Rₜ = 6.57 min, conversion 76 %, 27 % yield. ¹H-NMR (D₂O, 500 MHz) δ 6.06 (d, ³J = 4 Hz, 1H, 1-H^{I}), 5.44 (d, ³J = 2 Hz, 1H, 1-H^{II}), 5.20 (d, ³J = 1.5 Hz, 1H, 1-H^{III}), 4.44 (t, ³J = 5.75 Hz, 1H, 3-H^{II}), 4.39 (dd, ²J = 5 Hz, ³J = 2 Hz, 1H, 2-H^{II}), 4.26 (t, ³J = 3 Hz, 1H, 3-H^{III}), 4.24 (m, 1H, 4-H^{II}), 4.09 (t, ³J = 6.75 Hz, 1H, 5-H^{III}), 4.07 (m, 1H, 4-H), 4.01 (t, ³J = 10 Hz, 1H, 5-H^{I}), 3.98 - 3.92 (m, 3H, 5-H^{II}, 5-H, 3-H^{I}), 3.76 (dd, 1H, ²J = 12.5 Hz, ³J = 5.5 Hz, 5-H^{II}), 3.72- 3.68 (m, 2H, 4-H^{III}, 6-H), 3.60 (dd, ²J = 14 Hz, ³J = 7.5 Hz, 1H, 6a-H^{III}), 3.56 (m, 2H, 3-H, 2-H^{III}), 3.53-3.41 (m, 4H, 6a-H^{I}, 2-H^{I}, 6b-H^{III}, 4-H^{I}). 3.38 (m, 1H, 1-H), 3.32 (dd, ²J= 14 Hz, ³J = 6 Hz, 1H, 6b-H^{I}), 2.51 (dt, ²J = 12.5 Hz; ³J = 3.8 Hz, 1H, 2-He), 2.04 (s, 3H, CH₃), 1.89 (dd, ³J = ²J = 12.7 Hz, 1H, 2-Hₐ) ppm. APT (D₂O, 500 MHz) δ 174.49 (Carbonyl-C), 110.00 (C-1^{II}), 95.49 (C-1^{I}), 95.51 (C-1^{III}), 84.62 (C-5), 81.66 (C-4^{II}), 75.39 (C-3^{II}), 75.29 (C-4), 73.58 (C-2^{II}), 72.45 (C-5^{III}), 72.42 (C-6), 70.35 (C-4^{I}), 69.22 (C-5^{I}), 67.88 (C-3^{I}), 67.56 (C-3^{III}), 66.10 (C-4^{III}), 60.00 (C-5^{II}), 53.15 (C-2^{I}), 50.90 (C-2^{III}), 49.65 (C-1), 48.16 (C-3), 39.85 (C-6^{I}), 39.33 (C-6^{III}), 27.88 (C-2), 21.74 (CH₃) ppm, MS (EI+) *m*/*z*: 657.32739 [M+H]⁺. 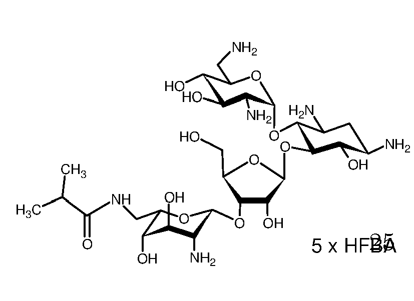 **N⁶(VI)-isobutyl neomycin B x 5 HFBA** (6). The title compound was prepared according to the general procedure described above. Derivative 6 was obtained as a white solid. For the measurement of regioselectivity and the characterization of the compound ¹H-NMR, HSQC as well as APT spectra were recorded and ESI-MS was employed. The yield was determined by HPLC: Rₜ = 9.65 min, conversion 65 %, 31 % yield. ¹H-NMR (D₂O, 500 MHz) δ 5.98 (s, 1H, 1-H^{I}), 5.38 (s, 1H, 1-H^{II}), 5.15 (s, 1H, 1-H^{III}), 4.39 (d, ³J = 6.0 Hz, 1H, 3-H^{II}), 4.37 (m, 1H, 2-H^{II}), 4.21 (m, 1H, 3-H^{III}), 4.17 (m, 1H, 4-H^{II}), 4.06-4.01 (m, 2H, 5-H^{III}, 4-H), 3.96 (t, ³J = 10.3 Hz, 1H, 5-H^{I}), 3.92 - 3.88 (m, 3H, 5-H^{II}, 5-H, 3-H^{I}), 3.70 (dd, 1H, ²J = 14 Hz, ³J= 5.8 Hz, 5-H^{II}), 3.65- 3.61 (m, 2H, 4-H^{III}, 6-H), 3.54-3.50 (m, 3H, 6a-H^{III}, 3-H, 2-H^{III}), 3.46 (m, 1H, 4-H^{I}), 3.44-3.38 (m, 3H, 6a-H^{I}, 2-H^{I}, 6b-H^{III}), 3.56-3.33 (m, 1H, 1-H), 3.28 (dd, ²J = 13.5 Hz, ³J = 6 Hz, 1H, 6b-H^{I}), 2.47 (m, 2H, CH (CH₃)₂, 2-Hₑ), 1.86 (dd, ³J = 12.2 Hz, 1H, 2-Hₐ), 1.09 (s, ³J = 6.5 Hz, 6H, CH₃) ppm. APT (D₂O, 500 MHz) δ 176.68 (carbonyl-C), 110.31 (C-1^{II}), 95.48 (C-1^{I}), 95.18 (C-1^{III}), 84.72 (C-5), 81.54 (C-4^{II}), 75.23 (C-4), 74.90 (C-3^{II}), 73.49 (C-2^{II}), 72.47 (C-5^{III}), 72.39 (C-6), 70.42 (C-4^{I}), 69.23 (C-5^{I}), 67.87 (C-3^{I}), 67.48 (C-3^{III}), 66.08 (C-4^{III}), 60.04 (C-5^{II}), 53.33 (C-2^{I}), 50.87 (C-2^{III}), 49.59 (C-1), 48.22 (C-3), 39.94 (C-6^{I}), 39.03 (C-6^{III}), 34.93 (CH(CH₃)₂), 27.95 (CH(CH₃)₂), 27.89 (C-2) ppm. MS (EI+) m/z: 685.36176 [M+H]⁺, 707.34387 [M+Na]⁺, 343.18356 [M+2H]²⁺. 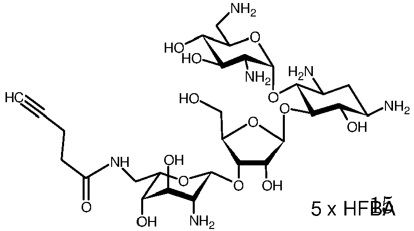 **N⁶(VI)-pent-4-inoyl neomycin B x 5 HFBA (7).** The title compound was prepared according to the general procedure described above. Derivative 7 was obtained as a white solid. For the measurement of regioselectivity and the characterization of the compound ¹H-NMR, HSQC as well as APT spectra were recorded and ESI-MS was employed. The yield was determined by HPLC: Rₜ = 9.8 min, conversion 83 %, 45 % yield. ¹H-NMR (D₂O, 500 MHz) δ 6.06 (d, ³J = 4 Hz, 1H, 1-H^{I}), 5.43 (s, 1H, 1-H^{II}), 5.19 (s, 1H, 1-H^{III}), 4.47 (d, ³J = 5.75 Hz, 1H, 3-H^{II}), 4.41 (m, 1H, 2-H^{II}), 4.26 (m, 1H, 3-H^{III}), 4.22 (m, 1H, 4-H^{II}), 4.11 - 4.06 (m, 2H, 5-H^{III} 4-H), 4.01 (t, ³J = 10 Hz, 1H, 5-H^{I}), 3.97 - 3.94 (m, 3H, 5-H^{II}, 5-H, 3-H^{I}), 3.77 (dd, ²J = 13 Hz. ³J = 5 Hz, 1H, 5-H^{II}), 3.75- 3.68 (m, 2H, 4-H^{III}, 6-H), 3.61 (dd, ²J = 14 Hz, ³J =7.5 Hz,1H, 6a-H^{III}), 3.56-3.53 (m, 2H, 3-H, 2-H^{III}), 3.52 - 3.45 (m, 4H, 4-H^{I}, 6a-H^{I}, 2-H^{I}, 6b-H^{III}), 3.36 (m, 1H, 1-H), 3.33 (dd, ²J = 14 Hz. ³J = 6 Hz, 1H, Gb-H^{I}), 2.56 - 2.43 (m, 6H. (CH₂)₂, 2-He, C≡C-H), 1.90 (dd, ³J = 12.3 Hz, 1H, 2-Hₐ) ppm. APT (D₂O, 500 MHz) δ 175.10 (carbonyl-C), 110.11 (C-1^{II}), 95.54 (C-1^{I}), 95.33 (C-1^{III}), 84.71 (C-5), 81.56 (C-4^{II}), 75.17 (C-3^{II}), 75.12 (C-4), 73.52 (C-2^{II}), 72.72 (C-5^{III}), 72.35 (C-6), 70.50 (C-4^{I}), 83.47 (C≡CH), 70.35 (C≡CH), 69.24 (C-5^{I}), 67.92 (C-3^{I}), 67.51 (C-3^{III}), 66.05 (C-4^{III}), 60.05 (C-5^{II}), 53.33 (C-2^{I}), 50.88 (C-2^{III}), 49.52 (C-1), 48.20 (C-3), 39.90 (C-6^{I}), 39.34 (C-6^{III}), 34.23 (CO-CH₂-CH₂), 27.80 (C-2), 14.54 (CO-CH₂-CH₂) ppm. MS (EI+) *m* /*z:* 695.34564 [M+H]⁺, 717.32770 [M+Na]⁺, 348.17706 [M+2H]²⁺. 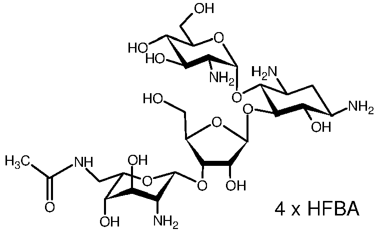 **N⁶(VI)-acetyl paromomycin x 4 HFBA (8).** The title compound was prepared according to the general procedure described above. Derivative 8 was obtained as a white solid. For the measurement of regioselectivity and the characterization of the compound ¹H-NMR, HSQC as well as APT spectra were recorded and ESI-MS was employed. The yield was determined by HPLC: Rₜ = 4.78 min, conversion 60 %, 22 % yield. ¹H-NMR (D₂O, 500 MHz) δ 5.81 (d, ³J = 3.5 Hz, 1H, 1-H^{I}), 5.39 (s, 1H, 1-H^{III}), 5.20 (s, 1H, 1-H^{III}), 4.44 (t, ³J = 5.5 Hz, 1H, 3-H^{III}), 4.36 (m, 1H, 1-H^{II}), 4.25 (m, 1H, 3-H^{III}), 4.22 (m, 1H, 4-H^{II}), 4.10 (t, ³J = 6.5 Hz, 1H, 5-H^{III}), 4.04 (t, ³J = 7.8 Hz, 1H, 4-H), 3.97- 3.89 (m, 4H, 6-H^{I}a, 5-H^{II}a, 3-H^{I}, 5-H), 3.81-3.75 (m, 3H, 6-H^{I}a, 5-H^{II}_{b}, 5-H^{I}), 3.73 - 3.67 (m, 2H, 4-H^{III}, 6-H), 3.62 - 3.57 (m, 2H, G-H^{III}ₐ, 3-H), 3.55 (m, 1H, 2-H^{III}), 3.51 (t, ³J = 9.3 Hz, 4-H^{I}), 3.43 (m, 1H, 2-H^{II}), 3.42 (dd, ²J= 15 Hz, ³J = 6 Hz, 1H, 6-H^{III}_{b}), 3.38-3.35 (m, 1H, 1-H), 2.51 (dt, ²J= 12,5 Hz. ³J = 2.5 Hz, 1H, 2-He), 2.04 (s, 3H, CH₃), 1.86 (dd, ³J = 12.5 Hz, 1H, 2-Hₐ) ppm. APT (D₂O, 500 MHz) δ 174.93 (carbonyl-C), 109.78 (C-1^{II}), 95.98 (C-1^{I}), 95.56 (C-1^{III}), 84.11 (C-5), 81.53 (C-4^{II}), 77.23 (C-4), 75.53 (C-3^{II}), 73.73 (C-5^{I}), 73.44 (C-2^{II}), 72.51 (C-5^{III}), 72.16 (C-6), 69.08 (C-4^{I}), 68.63 (C-3^{I}), 67.56 (C-3^{III}), 66.13 (C-4^{III}), 60.11 (C-6I, C-5^{II}), 53.161 (C-2^{I}), 50.88 (C-2^{III}), 49.48 (C-1), 48.63 (C-3), 39.38 (C-6^{III}), 27.87 (C-2), 21.71 (CH3) ppm. MS (EI+) *m*/*z*: 685.31303 m/z [M+H]+, 680.29454 [M+Na]⁺, 329.66013 [M+2H]²⁺.

### Antimicrobial tests

### Materials

Mueller Hinton II Broth powder (BD - cat. no. 212322)
Agar (Roth - cat. no. 5210.2)
6 mm paper disks (BBL - cat. no. 231039)

### Kirby-Bauer Test

*Antibiotic disks preparation.* Paper disks (6 mm diameter, BBL Microbiology Systems) were wetted through with 20, 25, 30 and 35 µL of a solution containing the antibiotic sample at a concentration of 0.5 nmol/µL. The wet disks were dried in a desiccator overnight, and used the next day.

*Culture preparation.* A colony picked from a freshly made plate of the bacteria strain *E. coli* ATCC 25922 was used to inoculate 20 mL of Mueller-Hinton broth and the obtained culture was grown overnight at 37 °C and 250 RPM shaking.

*Kirby-Bauer test procedure.* A new culture was made adding 1 mL of overnight culture in 99 mL of fresh Mueller-Hinton broth. The culture was grown at 37 °C and 250 RPM shaking until it reached an OD600 of 0.132 (0.5 McFarland) and a series of Mueller-Hinton-Agar plates preheated at 37 °C were inoculated spreading 200 µL of that culture with sterile cotton. The plates were then dried for 30 minutes. Then on each plate 3 or 4 antibiotic paper disks were placed. The plates were incubated overnight at 37 °C and subsequently the diameter of the inhibition growth zone was measured.

### Minimum Inhibitory Concentration (MIC) test

All test solutions contained 40 nmol of antibiotic. After drying, the samples were resuspended in 100 µL of Mueller-Hinton broth. Each solution of antibiotic in broth media was pipetted in the first lane of a 96 well plate (volume of well 500 µL). The remaining lanes were filled with 50 µL of *E. coli* ATCC 25922 culture with an OD600 of 0.264.

Series of 2-fold dilution were made removing from the first lane 50 µL of the antibiotic solution and resuspending it with the culture contained in the next well. At the end, all wells were filled with 100 µL of *E. coli* ATCC 25922 culture in Mueller-Hinton with an OD600 of 0.132 (0.5 McFarland) and the appropriate amount of antibiotic.

The 96-well plates were incubated overnight at 37 °C and 350 RPM shaking. The OD600 of all wells were measured using an *E. coli* ATCC 25922 culture with an OD600 of 0.132 as reference and the MIC value was determined by taking the lowest concentration where no bacterial growth was observed.

See also National Committee for Clinical Laboratory Standards, Performance Standards for antimicrobial susceptibility testing, 8th Informational Supplement (2002); John, D. T., et al. Antimicrobial Susceptibility testing: General Considerations, Manual of Clinical Microbiology 7th edition, 1469-1473 (1999); Laitha, M. K., Manual on antimicrobial susceptibility testing, 7-39 (2004).

### Example 2: Selection of Aminoglycoside -binding oligopeptides using phage display

This example discloses the selection of short peptide sequences capable of binding to Neomycin B and protecting the molecule partially to allow chemical reactions at the functional groups that are not involved in the host-guest interaction. The strategy to evolve peptide sequences that are able to recognize and bind to Neomycin B relies on the well established Phage Display technique (Smith GP. Science 1985; 228:1315-1317; Kay BK, et al. Gene 1993; 128:59-65).

For that purpose, the target molecule (Neomycin B) is covalently linked to a solid surface. In this case a Nunc Amino Immobilizer 96-wells plate was chosen as surface to couple Neomycin B. The plate contains in its wells a modified surface able to react with the amino-groups of the target molecule. The density of grafted complexes is approx. 10¹⁴ cm⁻².

In this way, the whole surface of the Neomycin B can interact with the peptide that is exposed on the recombinant phage particle, except for the site where it is covalently linked to the surface it is masked and therefore is not able to interact with the peptide.

### STEP 1 - IMMOBILIZATION OF NEOMYCIN B

The plate used (Nunc Immobilizer Amino F96 clear - Nunc #436006) is optimized for peptide and protein coupling. Several conditions were tested for the immobilization. Antibiotic binding was assayed by an ELISA test using an anti-neomycin antibody. The optimized protocol for the immobilization of the Neomycin is described in the following.

The coupling reaction was carried out by filling a well with 300µl of Neomycin B (1 mg/ml in sodium-phosphate 0.1M, pH 9.6). The plate was then placed on a shaker at 250 RPM at room temperature, for 12h. The well was then washed ten times using 300µl of TBST buffer (Tris-HCl 50mM + NaCl 0.15M pH 7.5 + 2% of Tween 20) and shaking 10 min at 250RPM and room temperature for each washing step.

### STEP 2 - PHAGE PANNING

Before starting the panning procedure the phages were incubated in an empty well to allow the "plastic binder" phages to be adsorbed on the plastic surface of the well. Therefore, 100 µl of a phage suspension containing 10¹² pfu/ml in sodium-phosphate 0.1M pH 7 were incubated in a blank well for 1h at room temperature shacked at 320 RPM. This procedure was repeated 3 times by taking the supernatant and putting it into another blank well. The final supernatant was used to perform the phage panning.

Three rounds of phage panning were carried out. Each round consists of 3 phases: incubation, washing and elution.

Incubation: 100µl/well of phage suspension were pipetted in each Neomycin B coated well and the plate was shaken gently for 60 min to allow the host-guest interaction to take place. The unbound phages were discarded by pouring off and slapping the plate face-down onto a clean paper towel.

Washing: each well was washed by adding 300µl of TBST and shaking for 10 min at room temperature. The unbound phages were discarded by pouring off and slapping the plate face-down onto a clean paper towel. The washing procedure was repeated 5 times during the first round of selection then 7 times during the second round and 10 times during the third one.

Elution: Sodium Phosphate 0.1M at pH 7.0 was used as elution buffer with the addition of Neomycin B at a concentration of 1µM as competitor to avoid non specific elution of phage particles.

### STEP 3 - SEQUENCING

Because of the use of Neomycin B as competitor, the cells *(E. coli* ER2738) were previously transformed with pET28 that carries the Kanamycin resistance that allows cell growth on Neomycin B containing media. Phage particles eluted after the third round of selection were used to infect ER2738 cells (already containing pET28). Subsequently, the cells were plated on LB-agar plate. After overnight incubation at 37°C several single colonies were picked and grown for 3h in 3 ml of LB medium. Single stranded DNA was then purified from the phage particles from the culture's supernatant using the QIAprep Spin M13 Kit (QIAGEN #27704) and prepared for sequencing.

The following peptide sequences were derived from the analysis of the DNA sequences V N R S S D H W N L T T, D Y D T L R T V A P T R, N G S L Q R S F V I S H, H V R I Y V D T I E I R, G A M H L P W H M G T L and G A M H P P R H M G P L .

### Example 3: Selection of Aminoglycoside-binding oligopeptides using in-vivo screening for small antibiotic binders.

An in-vivo screening for small antibiotic binders was performed by infecting a culture *of E. coli* ER2738 with a 12 amino acid random peptide-pIII phage library. The ability of the culture to survive under different antibiotic conditions was tested by measuring the OD600 after overnight growth.

A fresh culture of ER2838 was prepared by inoculation of 20 ml of LB medium with 50 µl glycerol stock solution of the strain. The culture (OD600 = 0.05) was infected with 100 µl of phage suspension containing 10¹³ pfu/ml with an overall complexity of 10⁹ different sequences. After 1 h of growth at 37°C shaking at 150-200 RPM the culture was aliquoted in 15 ml tubes resulting in a culture volume of 3 ml and the antibiotics at different concentrations were added.

The cultures were grown overnight at 37°C, shaking at 150-200 RPM. The OD600 of ten-fold diluted sample of each culture was estimated using a spectrophotometer. Not infected ER2738 and the same cells infected with a wild type M13 were used as negative control. The resistance against ampicillin, neomycin B and chloramphenicol was tested. It was observed that the cells infected with the library were more resistant in the presence of ampicillin and neomycin compared to the controls, but not in the case of chloramphenicol (data not shown). This demonstrates that the presence of the phage library influences the resistance of the cells at higher antibiotic concentration. The resistant cells were plated on LB-agar and the phage DNA was sequenced.

### EXAMPLE 4: Aptameric Protective Groups allow diverse modifications.

This example demonstrates that the APG approach for regioselective transformation according to the present invention is compatible with diverse reagents in different reactions. Scheme 1 shows the chemo- and regioselective transformation of ring IV of neomycin as exemplary target compound 1. All reactions were performed in 10 mM phosphate buffer at room temperature for 24 h in presence of 1.5 equiv. of APG **apt1.** (1) Acylation: 15 equiv. of 7, pH 6.9; (2) Thiolation: 5 equiv. of 9, pH 6.9; (3) Azide introduction: 8 equiv. of **11**, 0.36 mol % CuSO₄, Na₂CO₃, NaOH, pH 8.0; (4) Urea bond formation: 30 equiv. of **14a-c,** DMF, pH 6.9.

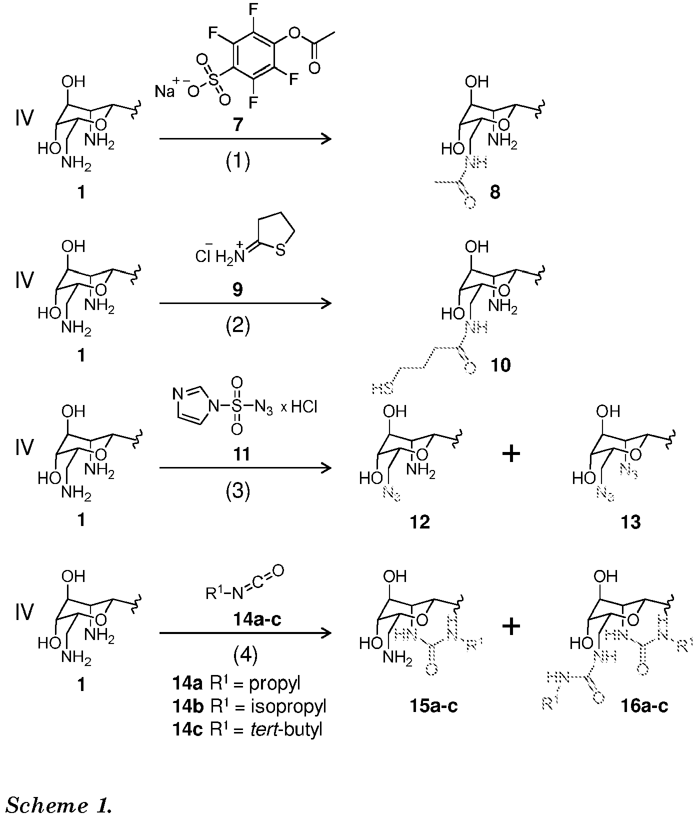

### General Procedures

### Synthesis of Sodium 4-(acetoxy)-2,3,5,6-tetrafluorobenzenesulfonate 7.

10.1 g of 2,3,5,6-tetrafluorophenol (61.4 mmol) was taken up in 22 mL fuming sulphuric acid (30 % SO3) and stirred at ambient temperature for 18h before pouring the mixture into 200 mL iced brine. The product was precipitated by adding 6 g of NaCl and stirred until no further precipitate was formed. This mixture was filtered through a sintered glass disc and the collected solids were taken up in 330 mL boiling acetonitril, filtered while hot, and allowed to cool slowly to ambient temperature. The colourless crystalline product was collected by filtration and dried in vacuum yielding 5.42 g (20.2 mmol, 33% yield) of 4-sulfo-tetrafluoro phenol sodium salt. 270 mg of this sodium salt (1.0 mmol) and 53.8 µL of acetic acid (0.94 mmol) were dissolved in 30 mL acetone. After 230 mg 1,3-dicyclohexylcarbodiimide (1.1 mmol) were added the mixture was stirred at room temperature for 20 hour. The resulting precipitate was removed by filtration and the filtrate was concentrated under reduced pressure. The crude mixture was purified by column chromatography using a 4:1 acetone/chloroform mixture. 163 mg (0.56 mmol, 56 % yield), white solid. Rt (acetone/chloroform 4:1) = 0.55. 1H-NMR (D2O, 400MHz): δ[ppm] = 2.46 (s, 3H, CH3-CO). 13C-NMR (D2O, 50.43 MHz): δ[ppm] = 170.07 (1C, CO); 147.01 (dq), 144.09 (ddd) (2C, 3-C-Ar, 5-C-Ar); 145.03 (dq), 142.10 (ddd) (2C, 2-CAr, 6-C-Ar), 131.14 (1C, C-SO2); 127.61 (1C, C-CO-CH3); 22.82 (1C, CH3-CO).

### Synthesis of diazo-transfer reagent Imidazole-1-sulfonyl Azide Hydrochloride 11.

Sulfuryl chloride (1.6 mL, 20 mmol) was added dropwise to an ice-cooled suspension of sodium azide (1.3 g, 20 mmol) in acetonitril (20 mL) and the mixture was stirred overnight at room temperature. Imidazole (2.6 g, 38 mmol) was added portion-wise to the ice-cooled mixture and the resulting slurry was stirred for additional 3 h at room temperature. The mixture was diluted with ethyl acetate (40 mL), washed with water (2 x 40 mL) and then with saturated aqueous sodium hydrogen carbonate (2 x 40 mL), dried over MgSO4 and filtered. The filtrate was cooled in an ice-batch and a 3M HCl methanolic solution (10mL) was added drop wise to precipitate the product. Finally, the filer cake was washed with EtOAc (3 x 10 mL) to obtain **11.** Yield: 1.9 g (9.1 mmol, 45% yield). 1H-NMR (D₂O, 400MHz) δ p.p.m.) 9.53 (s, 1H, H-2), 8.07 (s, 1H, H-5), 7.67 (s, 1H, H-4). 13C-NMR (D₂O, 400MHz) δ p.p.m.) 137.6, 122.6, 120.18. HRMS (EI+) *(m*/*z)*: found 174.0078 [M-Cl]+, calc. 174.0080 [M-Cl]+.

### (1) Acylation of amino group in C6 position of ring IV.

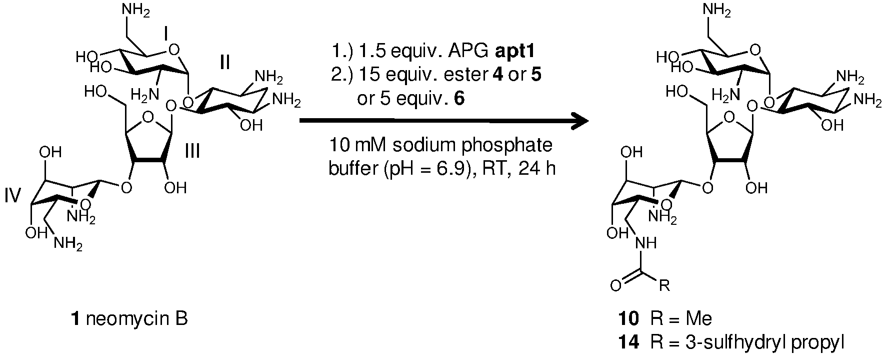

A volume of 900 µL of a 5.54 mM RNA aptamer solution (4.98 µmol) in 10 mM sodium phosphate buffer (pH 6.8) was heated to 85 °C for 10 min and was afterwards kept at room temperature for 15 min. 684 µL of a 4.8 mM solution of neomycin B sulphate (3.28 µmol) in 10 mM sodium phosphate buffer (pH 7.4) was added and the mixture was allowed to stand for 30 min at room temperature. Then, 15 equiv. activated ester, acetyl sulfo-NHS ester 4 or STP-ester 5 (49.2 µmol) 500 µL 10 mM sodium phosphate buffer (pH 7.4), or 5 equiv. 2-iminothiolane hydrochloride (16.4 µmol) dissolved in 42 µL 10 mM sodium phosphate buffer (pH 7.4) were added and the reaction mixture was allowed to react for 24 hours at room temperature. After addition of 180 µL of a 7 wt. % ethylamine water solution and further incubation for 30 min at room temperature, 486 µl of a 2 M sodium hydroxide solution were added and the crude mixture was heated to 90 °C for 30 min. After cooling to room temperature each 50 µL fraction was purified by HPLC using a *Waters* Spherisorb ODS-2C₁₈ analytic column (water/acetone 1:0.81 containing 16.9 mM HFBA) at a flow rate of 1 ml/min at 40 °C to afford the antibiotic derivatives **10** and **14.** After evaporation of acetone and freeze-drying of collected fractions the product was taken up in 150 µL of D₂O for NMR-studies.

### (2) Urea Bond Formation at 2C & 6C Position

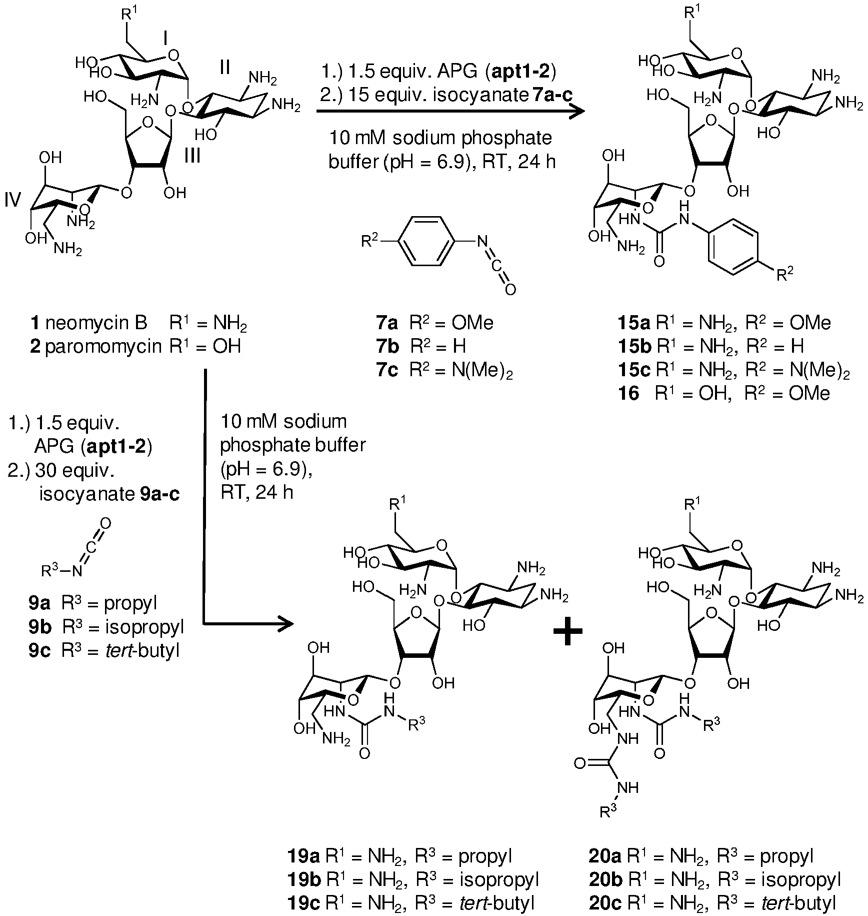

900 µL of a 5.54 mM RNA aptamer solution (4.98 µmol) in 10 mM sodium phosphate buffer (pH 6.8) was heated to 85 °C for 10 min and was afterwards kept at room temperature for 15 min. 684 µL of a 4.8 mM solution of the aminoglycoside antibiotic (3.28 µmol) in 10 mM sodium phosphate buffer (pH 7.4) was added and the mixture was allowed to stand for 30 min at room temperature. 15 equiv. (49.2 µmol) aromatic isocyanate **7a-c** or 30 equiv. (98.4 µmol) of aliphatic isocyanates **9a-c** dissolved in 108 µL DMF were added and the reaction mixture was allowed to react for 24 hours at room temperature. After addition of 180 µL of a 7 wt. % ethylamine water solution and further incubation for 30 min at room temperature, 280 µl of a 2 M sodium hydroxide solution was added and the crude mixture was heated to 90 °C for 30 min. After cooling to room temperature each 50 µL fraction was purified by HPLC using a *Waters* Spherisorb ODS-2C₁₈ analytic column (water/acetone 1.0:0.81 containing 16.9 mM HFBA) at a flow rate of 1 ml/min at 40 °C to afford the antibiotic derivatives **15a-c, 16, 19a-c** and **20a-b.** After evaporation of acetone and freeze-drying the product was taken up in 150 µL of D₂O for NMR-studies.

### (3) Azide Introduction at 2C and 6C Position

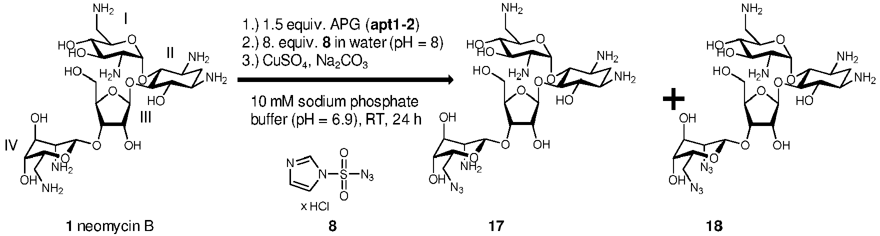

900 µL of a 5.54 mM RNA aptamer solution (4.98 µmol) in 10 mM sodium phosphate buffer (pH 6.8) was heated to 85 °C for 10 min and was afterwards kept at room temperature for 15 min. 684 µL of a 4.8 mM solution of neomycin B sulphate (3.28 µmol) in 10 mM sodium phosphate buffer (pH 7.4) was added and the mixture was allowed to stand for 30 min at room temperature. 540 µL of an 4.8 mM aqueous

solution of diazo-transfer reagent 8 (10 mg/mL), which was adjusted to pH 8 by approx. 25 µL of adding 2 M NaOH solution, was added into the solution of the antibiotic **1 - apt1** complex solution. After 59 µL of an aqueous solution of sodium carbonate (10 mg/mL) and 50 µL of an aqueous solution of copper sulfate (2 mg/mL) were added and the mixture was reacted for 24 hours at room temperature. After addition of 180 µL of a 7 wt. % ethylamine water solution and further incubation for 30 min at room temperature, 375 µl of a 2 M sodium hydroxide solution was added and the crude mixture was heated to 90 °C for 30 min. After cooling to room temperature each 50 µL fraction was purified by HPLC using a *Waters* Spherisorb ODS-2C₁₈ analytic column (water/acetone 1.0:0.81 containing 16.9 mM HFBA) at a flow rate of 1 ml/min at 40 °C to afford the antibiotic derivatives 17 and 18. After evaporation of acetone and freeze-drying of collected fractions the product was taken up in 150 µL of D₂O for NMR-studies.

### REFERENCES

1. Alper, P. B., Hendrix, M., Sears, P., Wong, C.-H. Probing the specificity of aminoglycoside ribosomal RNA interactions with designed synthetic analogs. J. Am. Chem. Soc. 120, 1956-1978 (1998).
2. Usui, T., Umezawa, S. Total synthesis of neomycin B, Carbohydrate Research 174, 133-143 (1988).
3. Hanessian, S. et at.6-Hydroxy to 6"'-amino tethered ring-to-ring macrocyclic aminoglycosides as probes for APH(3')-IIIa kinase, Bioorg. & Med. Chem. Lett. 17, 3221-3225 (2007).
4. Coquire, D., de la Lande, A., Parisel, O., Prang, T., Reinaud, O. Directional control and supramolecular protection allowing the chemo and regioselective Transformation of a Triamine. Chem. Eur. J. 15, 11912 - 11917 (2009).
5. Cafeo, G., Kohnke, F. H., Valenti, L., Cafeo, G. et al. Regioselective O-alkylations and acylations of polyphenolic substrates using a calix[4]pyrrole derivative. Tetrahedron Letters 50, 2009, 4138-4140 (2009).
6. Sun, W., Du, L., Li, M. Aptamer-based carbohydrate recognition. Current Pharmaceutical Design 16, 2269-2278 (2010).
7. Beta, H. et al. Aptamers that recognize the lipid moiety of the antibiotic moenomycin A. Biol. Chem. 284, 1497-1500 (2003).
8. Jiang, L., Suri, A. K., Fiala, R., Patel, D. J. Saccharide-RNA recognition in an aminoglycoside antibiotic-RNA aptamer complex. Chemistry & Biology 4, 35-50 (1997).
9. Wang, K. Y., McCurdy, S., Shea, R. G., Swaminathan, S., Bolton, P. H. A DNA aptamer which bind to and inhibits thrombin exhibits a new structural motif for DNA. Biochemistry 32, 1899-1904 (1993).
10. Wallis, M. G., von Ahsen, U., Schroeder, R., Famulok, M. A novel RNA motif for neomycin recognition. Chemistry and Biology 2, 543-552 (1995).
11. Connell, G. J., Illangesekare, M., Yarus, M. Three small ribooligonucleotides with specific arginine sites , Biochemistry 32, 5497-5502 (1993).
12. Sassanfar, M., Szostak, J. W. An RNA motif that binds ATP, Nature 364, 550-553 (1993).
13. Huizenga, D. E., Szostak, J. W. Identification of N2-(1-Carboxyethyl)guanine (CEG) as a Guanine Advanced Glycosylation End Product, Biochemistry 34, 656-665 (1995).
14. Burke, D. H., Hoffmann, D. C. A novel acidophilic RNA motif that recognizes Coenzyme A, Biochemistry 37, 4653-4663 (1998).
15. Ellington, A. D., Szostak, J. W. Selection in citro of single-stranded DNA molecules that fold into specific ligand-binding structures, Nature 355, 850-852 (1992).
16. Li, Y. F., Geyer, C. R. Sen, D. Recognition of anionic porphyrins by DNA aptamers, Biochemistry 35, 6911-6922 (1996).
17. Wang, C. Y. E., Su, W. P. D., Kurtin, P. J. Subcutaneous panniculitic T-Cell Lymphoma, Int. J. Dermat. 35, 1-8 (1996).
18. Moazed, D., Noller, H. F. Interaction of antibiotics which functional sites in 16S ribosomal RNA. Nature 327, 389-394 (1987).
19. Purohi, P., Stern, S. Interaction of small RNA with antibiotics and RNA ligands to the 30S subunit. Nature 370, 659-662 (1994).
20. Zhou, J.,Wang, G., Zhang, L.-H., Ye, X.-S. Modification of aminoglycoside antibiotics targeting RNA. Med. Res. Rev. 27, 279-316 (2007).
21. Samantaray, S., Marathe, U., Dasgupta, S., Nandicoori, V. K., Roy, R. P. Peptide-sugar ligation catalyzed by transpeptidase sortase: a facile approach to neoglycoconjugate synthesis. J. Am. Chem. Soc. 130, 2132-2133 (2009).
22. Chang, C.-W. T. et al. Surprising alteration of antibacterial activity of 5"-modified neomycin against resistant bacteria. J. Med. Chem. 51, 7563-7573 (2008).
23. Kirk, S. R., Tor, Y. tRNAPhe binds aminoglycoside antibiotics. Bioorg. & Med. Chem. 7, 1979-1991 (1999).
24. Tok, J. B.-H., Fenker, J. Novel synthesis and RNA-binding properties of aminoglycoside dimers conjugated via a naphthalene diimide-based intercalator. Bioorg. & Med. Chem. Lett. 11, 2987-2991 (2001).
25. Bera, S., Zhanel, G. G., Schweizer, F. Evaluation of amphiphilic aminoglycoside-peptide triazole conjugates as antibacterial agents. Bioorg. & Med. Lett. 20, 3031-3035 (2010).
26. François, B. et al. Crystal structures of complexes between aminoglycosides and decoding A site oligonucleotides: role of the number of rings and positive charges in the specific binding leading to miscoding. Nucleic Acids 33, 5677-5690 (2005).
27. Mikkelsen,, N. E., Johansson, K., Virtanen, A., Kirsebom, L.A. Aminoglycosides binding displaces a divalent metal ion in a tRNA-neomycin B complex. Nat. Struct. Biol. 8, 510-514 (2001).
28. Stampfl, S., Lempradl, A., Koehler, G. Schroeder, R. Monovalent ion dependence of neomyin B binding to an RNA aptamer characterized by spectroscopic methods. Chem. Bio. Chem. 8, 1137-1145 (2007).
29. Jiang, L. et al. Saccharide-RNA recognition in a complex formed between neomycin B and a RNA aptamer. Structure 7, 817-827 (1999).

## Claims

1. A method for the chemo- and/or regioselective derivatization of a target compound comprising multiple chemically equivalent reactive groups, wherein at least one reactive group is to be derivatized and wherein at least one reactive group is not to be derivatized, the method comprising the steps of
a. contacting the target compound with at least one non-covalent protective group under conditions allowing for the formation of a regioselective host-guest complex, wherein the target compound is an aminoglycoside antibiotic based on a neamine scaffold and wherein the protective group is a DNA or RNA aptamer or oligopeptide aptamer having a selective affinity for the at least one reactive group not to be modified, wherein the RNA or DNA aptamer consists of from 8 to 60 nucleotides and is obtained by a screening process comprises the steps of: (1) constructing a random single-stranded DNA (ssDNA) library and preparing a primer; (2) preparing the random single-stranded DNA (ssDNA) library by PCR amplification (for DNA SELEX) or an RNA library by transcription (for RNA SELEX); (3) carrying out multiple rounds of SELEX screening; (4) detecting the appetency; (5) cloning and sequencing DNA, or wherein the oligopeptide aptamer is obtained by expressing a library of candidate oligopeptide aptamers in a recombinant host cell by infection with phages, selecting at least one host cell expressing an oligopeptide aptamer, and identifying the oligopeptide aptamer; followed and/or accompanied by
b. chemical derivatization of the target compound.

2. Method according to claim 1, wherein the aminoglycoside antibiotic is selected from the group consisting of neomycin, paromomycin, ribostamycin, kanamycin and streptomycin.

3. Method according to any one of the preceding claims, wherein the RNA or DNA aptamer consists of from 15 to 40 nucleotides.

4. Method according to claim 3, wherein the RNA aptamer is 5'-GGA CUG GGC GAG AAG UUU AGU CC-3', 5'-CUG CAG UCC GAA AAG GGC CAG-3', 5'- UGU GUA GGG CGA AAA GUU UUA-3' or 5'-GGC ACG AGG UUU AGC UAC ACU CGU GCC-3'.

5. Method according to claim 1 or 2, wherein the protective group is an oligopeptide aptamer, preferably wherein the oligopeptide aptamer consists of from 8-18 amino acids, preferably 10-13 amino acids.

6. Method according to claim 5, wherein oligopeptide aptamer is selected from the group consisting of VNRSSDHWNLTT, DYDTLRTVAPTR, NGSLQRSFVISH, HVRIYVDTIEIR, GAMHLPWHMGTL and GAMHPPRHMGPL.

7. Method according to any one of the preceding claims, wherein the chemical derivatization comprises acylation, alkylation, oxidation, PEGylation, reductive amination, aza-Michael reaction or urea bond formation.

8. An aminoglycoside antibiotic derivative, having the general formula

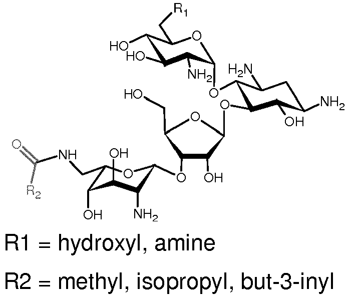

9. An aminoglycoside antibiotic derivative having the general formula

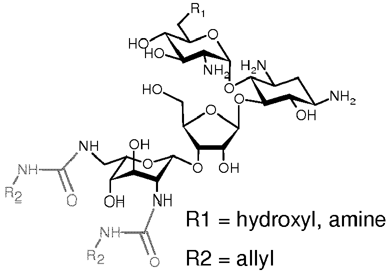

10. An aminoglycoside antibiotic derivative, selected from the group consisting of N⁶(IV) acetyl neomycin B (Formula 1), N⁶(IV) dimethylacetyl neomycin B (Formula 2), N⁶(IV) pent-3-inoyl neomycin B (Formula 3), N²(IV) {[(phenyl)amino]carbonyl}amino neomycin B (Formula 4), N²(IV) {[(4-methoxyphenyl)amino]carbonyl} amino neomycin B (Formula 5), N²(IV) {[(4-methoxyphenyl)amino]carbonyl}amino paromomycin (Formula 6) and N²(IV),N⁶(IV)-bis{[(allylamino)carbonyl]amino}neomycin B (Formula 7), N⁶(IV) acetyl paromomycin, N⁶(IV)-γ-sulfhydryl-propionyl neomycin B, N⁶-(IV)-azido neomycin B, N⁶-(IV), N²-(IV)-diazido neomycin B, N²(IV)-(propylamino)carbonyl neomycin B, N²(IV)-(isopropylamino)carbonyl neomycin B, N²(IV)-(tert-butylamino)carbonyl neomycin B, N²(IV), N⁶(IV)-bis-N-(propylamino)carbonyl neomycin B, N²(IV), N⁶(IV)-bis-N-(isopropylamino)carbonyl neomycin B, N²(IV), N⁶(IV)-bis-N-(*tert*-butylamino)carbonyl neomycin B, N⁶(II)-γ-sulfhydryl butanoyl neomycin B and pharmaceutically acceptable salts thereof.

11. A kanamycin derivative selected from the group consisting of N⁶(II)-acetyl kanamycin A or B, N⁶(II)-2-methylpropionyl kanamycin A or B, N⁶(II)-2-butynyl kanamycin A or B or N⁶(II)-γ-sulfhydryl butanoyl kanamycin A or B, and pharmaceutically acceptable salts thereof.

12. A pharmaceutical composition comprising an aminoglycoside antibiotic derivative according to any one of claims 8 to 11.

13. An aminoglycoside antibiotic derivative according to any one of claims 8 to 11 for use as a medicament.

14. An aminoglycoside antibiotic derivative according any one of claims 8 to 11 for use in a method of treatment of a bacterial infection, preferably an infection with Methicillin-resistant *Staphylococcus aureus* (MRSA) or vancomycin-resistant enterococci (VRE).

15. Compound according to any one of claims 8 to 11 for use as antimicrobial agent.

## Patentansprüche

1. Verfahren zur chemo- und/oder regioselektiven Derivatisierung einer Zielverbindung, umfassend mehrere chemisch äquivalente reaktive Gruppen, wobei mindestens eine reaktive Gruppe zu derivatisieren ist und wobei mindestens eine reaktive Gruppe nicht zu derivatisieren ist, das Verfahren umfassend die Schritte von:
a. Kontaktieren der Zielverbindung mit mindestens einer nicht kovalenten Schutzgruppe unter Bedingungen, die die Bildung eines regioselektiven Wirt-Gast-Komplexes erlauben, wobei die Zielverbindung ein Aminoglykosid-Antibiotikum basierend auf einem Neamingerüst ist und wobei die Schutzgruppe ein DNA- oder RNA-Aptamer oder Oligopeptid-Aptamer mit einer selektiven Affinität für die mindestens eine nicht zu modifizierende reaktive Gruppe ist, wobei das RNA- oder DNA-Aptamer aus von 8 bis 60 Nukleotiden besteht und erhalten wird durch einen Screening-Vorgang, umfassend die Schritte von: (1) Aufbauen einer zufälligen einzelsträngigen DNA (ssDNA)-Bibliothek und Vorbereiten eines Primers; (2) Vorbereiten der zufälligen einzelsträngigen DNA (ssDNA)-Bibliothek durch PCR-Amplifikation (für DNA SELEX) oder einer RNA-Bibliothek durch Transkription (für RNA SELEX); (3) Ausführen mehrerer Durchgänge von SELEX-Screening; (4) Erkennen der Appetenz; (5) Klonieren und Sequenzieren von DNA, oder wobei das Oligopeptid-Aptamer erhalten wird durch Exprimieren einer Bibliothek von Kandidat-Oligopeptid-Aptameren in einer rekombinanten Wirtszelle durch Infektion mit Phagen, Auswählen von mindestens einer Wirtszelle, die ein Oligopeptid-Aptamer exprimiert, und Identifizieren des Oligopeptid-Aptamers; gefolgt und/oder begleitet von
b. chemischer Derivatisierung der Zielverbindung.

2. Verfahren nach Anspruch 1, wobei das Aminoglykosid-Antibiotikum ausgewählt ist aus der Gruppe bestehend aus Neomycin, Paromomycin, Ribostamycin, Kanamycin und Streptomycin.

3. Verfahren nach einem der vorhergehenden Ansprüche, wobei das RNA- oder DNA-Aptamer aus von 15 bis 40 Nukleotiden besteht.

4. Verfahren nach Anspruch 3, wobei das RNA-Aptamer 5'-GGA CUG GGC GAG AAG UUU AGU CC-3', 5'-CUG CAG UCC GAA AAG GGC CAG-3', 5'-UGU GUA GGG CGA AAA GUU UUA-3' oder 5'-GGC ACG AGG UUU AGC UAC ACU CGU GCC-3' ist.

5. Verfahren nach Anspruch 1 oder 2, wobei die Schutzgruppe ein Oligopeptid-Aptamer ist, bevorzugt wobei das Oligopeptid-Aptamer aus 8-18 Aminosäuren, bevorzugt 10-13 Aminosäuren, besteht.

6. Verfahren nach Anspruch 5, wobei das Oligopeptid-Aptamer ausgewählt ist aus der Gruppe, bestehend aus VNRSSDHWNLTT, DYDTLRTVAPTR, NGSLQRSFVISH, HVRIYVDTIEIR, GAMHLPWHMGTL und GAMHPPRHMGPL.

7. Verfahren nach einem der vorhergehenden Ansprüche, wobei die chemische Derivatisierung Acylierung, Alkylierung, Oxidation, PEGylierung, reduktive Aminierung, Aza-Michael-Reaktion oder die Bildung von Harnstoffbindung umfasst.

8. Aminoglykosid-Antibiotikum-Derivat mit der allgemeinen Formel 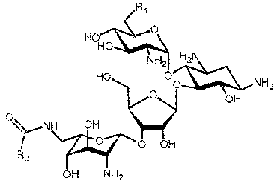
R1 = Hydroxyl, Amin
R2 = Methyl, Isopropyl, But-3-inyl

9. Aminoglykosid-Antibiotikum-Derivat mit der allgemeinen Formel

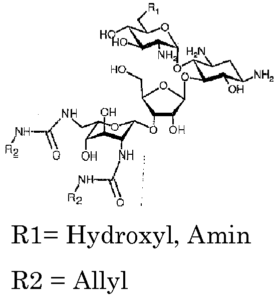

10. Aminoglykosid-Antibiotikum-Derivat, ausgewählt aus der Gruppe, bestehend aus N⁶(IV)-Acetylneomycin B (Formel I), N⁶(IV)-Dimethylacetylneomycin B (Formel 2), N⁶(IV)-Pent-3-inoylneomycin B (Formel 3), N²(IV)-{[(Phenyl)amino]carbonyl}aminoneomycin B (Formel 4), N²(IV)-{[(4-Methoxyphenyl)amino]carbonyl}aminoneomycin B (Formel 5), N²(IV)-{[(4-Methoxyphenyl)amino]carbonyl}aminoparomomycin (Formel 6) und N²(IV),N⁶(IV)-Bis{[(allylamino)carbonyl]amino}neomycin B (Formel 7), N⁶(IV)-Acetylparomomycin, N⁶(IV)-γ-Sulfhydrylpropionylneomycin B, N⁶(IV)-Azidoneomycin B, N⁶(IV),N²(IV)-Diazidoneomycin B, N²(IV)-(Propylamino)carbonylneomycin B, N²(IV)-(Isopropylamino)carbonylneomycin B, N²(IV)-(*tert*-Butylamino)carbonylneomycin B, N²(IV),N⁶(IV)-Bis-N-(propylamino)carbonylneomycin B, N²(IV),N⁶(IV)-Bis-N-(isopropylamino)carbonylneomycin B, N²(IV), N⁶(IV)-Bis-N-(*tert-*butylamino)carbonylneomycin B, N⁶(II)- γ-Sulfhydrylbutanoylneomycin B und pharmazeutisch verträgliche Salze davon.

11. Kanamycin-Derivat, ausgewählt aus der Gruppe bestehend aus N⁶(II)-Acetylkanamycin A oder B, N⁶(II)-2-Methylpropionylkanamycin A oder B, N⁶(II)-2-Butynylkanamycin A oder B oder N⁶(II)-γ-Sulfhydrylbutanoylkanamycin A oder B und pharmazeutisch verträgliche Salze davon.

12. Pharmazeutische Zusammensetzung, umfassend ein Aminoglykosid-Antibiotikum-Derivat nach einem der Ansprüche 8 bis 11.

13. Aminoglykosid-Antibiotikum-Derivat nach einem der Ansprüche 8 bis 11 zur Verwendung als ein Arzneimittel.

14. Aminoglykosid-Antibiotikum-Derivat nach einem der Ansprüche 8 bis 11 zur Verwendung in einem Verfahren zur Behandlung einer bakteriellen Infektion, bevorzugt einer Infektion mit Methicillin-resistentem *Staphylococcus aureus* (MRSA) oder Vancomycin-resistenten Enterokokken (VRE).

15. Verbindung nach einem der Ansprüche 8 bis 11 zur Verwendung als antimikrobieller Wirkstoff.

## Revendications

1. Procédé de dérivatisation chimio- et/ou régiosélective d'un composé cible comprenant de multiples groupes réactifs chimiquement équivalents, dans lequel au moins un groupe réactif doit être dérivatisé et dans lequel au moins un groupe réactif ne doit pas être dérivatisé, le procédé comprenant les étapes suivantes
a) mise en contact du composé cible avec au moins un groupe protecteur non covalent dans des conditions permettant la formation d'un complexe hôtelier-client régiosélectif, dans lequel le composé cible est un antibiotique aminoglycoside basé sur un échafaudage de néamine et dans lequel le groupe protecteur est un aptamère d'ADN et d'ARN ou un aptamère oligopeptidique présentant une affinité sélective vis-à-vis de l'au moins un groupe réactif à ne pas modifier, dans lequel l'aptamère d'ARN ou d'ADN est constitué de 8 à 60 nucléotides et est obtenu par un procédé de criblage, comprenant les étapes suivantes: (1) construction d'une banque d'ADN monocaténaire aléatoire (ADNsb) et préparation d'une amorce ; (2) préparation de la banque d'ADN monocaténaire aléatoire (ADNsb) par amplification PCR (pour la méthode SELEX ADN) ou d'une banque d'ARN par transcription (pour la méthode SELEX ARN) ; (3) réalisation de plusieurs cycles de criblage SELEX ; (4) détection de l'appétence ; (5) clonage et séquençage de l'ADN, ou dans lequel l'aptamère oligopeptidique est obtenu par expression d'une banque d'aptamères oligopeptidiques candidats dans une cellule hôte recombinante par infection avec des phages, sélection d'au moins une cellule hôte exprimant un aptamère oligopeptidique, et identification de l'aptamère oligopeptidique ; suivie et/ou accompagnée de la
b) dérivatisation chimique du composé cible.

2. Procédé selon la revendication 1, dans lequel l'antibiotique aminoglycoside est choisi dans le groupe constitué de la néomycine, de la paromomycine, de la ribostamycine, de la kanamycine et de la streptomycine.

3. Procédé selon l'une quelconque des revendications précédentes, dans lequel l'aptamère d'ARN ou d'ADN est constitué de 15 à 40 nucléotides.

4. Procédé selon la revendication 3, dans lequel l'aptamère d'ARN est 5'-GGA CUG GGC GAG AAG UUU AGU CC-3', 5'-CUG CAG UCC GAA AAG GGC CAG-3', 5'-UGU GUA GGG CGA AAA GUU UUA-3' ou 5'-GGC ACG AGG UUU AGC UAC ACU CGU GCC-3'.

5. Procédé selon la revendication 1 ou 2, dans lequel le groupe protecteur est un aptamère oligopeptidique, de préférence dans lequel l'aptamère oligopeptidique est constitué de 8 à 18 acides aminés, de préférence de 10 à 13 acides aminés.

6. Procédé selon la revendication 5, dans lequel l'aptamère oligopeptidique est choisi dans le groupe constitué de VNRSSDHWNLTT, DYDTLRTVAPTR, NGSLQRSFVISH, HVRIYVDTIEIR, GAMHLPWHMGTL et GAMHPPRHMGPL.

7. Procédé selon l'une quelconque des revendications précédentes, dans lequel la dérivatisation chimique comprend l'acylation, l'alkylation, l'oxydation, la PEGylation, l'amination réductrice, la réaction d'aza-Michael ou la formation de liaisons urée.

8. Dérivé d'antibiotique aminoglycoside, répondant à la formule générale

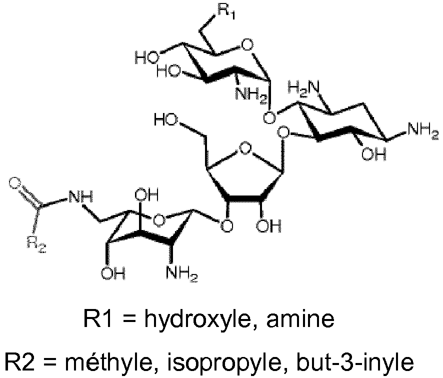

9. Dérivé d'antibiotique aminoglycoside répondant à la formule générale

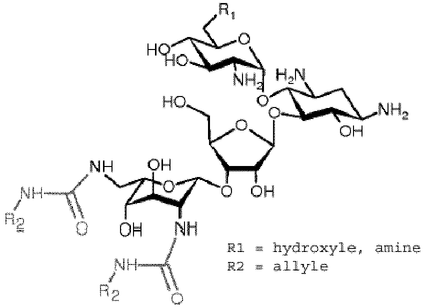

10. Dérivé d'antibiotique aminoglycoside, choisi dans le groupe constitué de N⁶(IV) acétyl néomycine B (Formule 1), N⁶(IV) diméthylacétyl néomycine B (Formule 2), N⁶(IV) pent-3-inoyl néomycine B (Formule 3), N²(IV) {[(phényl)amino]carbonyl}amino néomycine B (Formule 4), N²(IV) {[(4-méthoxyphényl)amino]carbonyl} amino néomycine B (Formule 5), N²(IV) {[(4-méthoxyphényl)amino]carbonyl}amino paromomycine (Formule 6) et N²(IV),N⁶(IV)-bis{[(allylamino)carbonyl]amino} néomycine B (Formule 7), N⁶(IV) acétyl paromomycine, N⁶(IV)-γ-sulfhydryl-propionyl néomycine B, N⁶-(IV)-azido néomycine B, N⁶-(IV), N²-(IV)-diazido néomycine B, N²(IV)-(propylamino)carbonyl néomycine B, N²(IV)-(isopropylamino)carbonyl néomycine B, N²(IV)-(tert-butylamino)carbonyl néomycine B, N²(IV), N⁶(IV)-bis-N-(propylamino)carbonyl néomycine B, N²(IV), N⁶(IV)-bis-N-(isopropylamino)carbonyl néomycine B, N²(IV), N⁶(IV)-bis-N-(*tert*-butylamino)carbonyl néomycine B, N⁶(II)-γ-sulfhydryl butanoyl néomycine B et des sels pharmaceutiquement acceptables de celles-ci.

11. Dérivé de kanamycine choisi dans le groupe constitué de N⁶(II)-acétyl kanamycine A ou B, N⁶(II)-2-méthylpropionyl kanamycine A ou B, N⁶(II)-2-butynyl kanamycine A ou B ou N⁶(II)-γ-sulfhydryl butanoyl kanamycine A ou B, et des sels pharmaceutiquement acceptables de celles-ci.

12. Composition pharmaceutique comprenant un dérivé d'antibiotique aminoglycoside selon l'une quelconque des revendications 8 à 11.

13. Dérivé d'antibiotique aminoglycoside selon l'une quelconque des revendications 8 à 11 pour utilisation en tant que médicament.

14. Dérivé d'antibiotique aminoglycoside selon l'une quelconque des revendications 8 à 11 pour utilisation dans un procédé de traitement d'une infection bactérienne, de préférence d'une infection par *Staphylococcus aureus* résistant à la méthicilline (MRSA) ou par les entérocoques résistants à la vancomycine (VRE).

15. Composé selon l'une quelconque des revendications 8 à 11 pour utilisation en tant qu'agent antimicrobien.
